# EUROPÄISCHE PATENTANMELDUNG

(11) **EP 2 481 392 A2**
(43) Veröffentlichungstag der Anmeldung: **01.08.2012**
(21) Anmeldenummer: 12152060.5
(22) Anmeldetag: 23.01.2012
(51) Int. Cl.: A61K 8/31, A61K 8/37, A61K 8/67, A61Q 15/00

(54) **Hautnährende Antitranspirant-Zusammensetzungen enthaltend Squalan und Tocopherol(ester)**

(30) Priorität: 28.01.2011 DE 102011003331
(71) Anmelder: Henkel AG & Co. KGaA, 40589 Düsseldorf (DE)
(72) Erfinder: Banowski, Bernhard, 40597 Düsseldorf (DE); Heide, Barbara, 47809 Krefeld (DE); Holtkötter, Olaf, 50354 Hürth (DE)

(57) **Zusammenfassung**

Die vorliegende Erfindung betrifft Antitranspirant-Zusammensetzungen mit besonders hoher Haut pflegender und nährender Wirkung, die eine die Synthese von Adenosintriphosphat (ATP) in den Hautzellen stimulierende Wirkstoffkombination aus Squalan, weiterhin mindestens einer Substanz, ausgewählt aus Tocopherol und Tocopherylestern sowie Mischungen hiervon, sowie mindestens einem Glycerindiester oder Glycerintriester von mindestens einer gesättigten, unsubstituierten Alkansäure mit 8 bis 18 Kohlenstoffatomen enthält.

## Beschreibung

Die vorliegende Erfindung betrifft Antitranspirant-Zusammensetzungen mit besonders hoher Haut pflegender und nährender Wirkung.

Handelsübliche Antitranspirant-Zusammensetzungen enthalten als Antitranspirant-Wirkstoff mindestens ein wasserlösliches adstringierendes anorganisches und/oder organisches Salz des Aluminiums, Zinks oder ausgewählte Aluminium-Zirkonium-Mischsalze. Antitranspirant-Zusammensetzungen werden üblicherweise im Bereich der Achselhöhlen appliziert und angewendet. Die Achselhaut weist aufgrund ihrer Lage und Funktion eine schwache Barrierefunktion auf. Die Haut im Achselbereich ist also empfindlicher als die restliche Haut des Körpers, häufig mindestens genau so empfindlich wie die Haut des Gesichtes. Viele Verbraucher betrachten eine unbehaarte Achselhaut als ästhetischer und der Hygiene zuträglicher, denn der Haarwuchs vergrößert die schweißtragende Oberfläche im Achselbereich und trägt damit zu einer verstärkten Körpergeruchsentwicklung bei. Dementsprechend gehört eine regelmäßige Rasur der Achselbehaarung für viele Verbraucher zur Hygieneroutine dazu. Durch die Achselrasur wird die Haut mechanisch gereizt und die Barrierefunktion zusätzlich geschwächt. Nach der Rasur wird auf die Achselhaut üblicherweise eine kosmetische Antitranspirant-Zusammensetzung appliziert, beispielsweise als Spray oder mit einem Roll-on oder Stift. Die schweißhemmenden Wirkstoffe weisen, insbesondere wegen ihres sauren pH-Wertes, häufig ein beträchtliches Reizpotenzial auf, was auf der Achselhaut Hautrötungen, ein unangenehmes Brennen, Spannungsgefühl und/oder Jucken hervorrufen kann.

Das Adenosintriphosphat (ATP) ist ein zentrales Molekül des Energiehaushalts lebender Zellen, da es die Energiequelle vieler zellulärer Reaktionen darstellt. Der intrazelluläre ATP-Gehalt ist ein sensitiver Marker für die Vitalität und Aktivität von Zellen, da eine erhöhte zelluläre Aktivität, z. B. während der Proliferation (Zellvermehrung), mit einem erhöhten Bedarf und Verbrauch von ATP korreliert. Insofern ist ein hoher Gehalt an ATP ein Zeichen einer hohen Leistungsfähigkeit der Zellen, was sich im hohen ATP-Gehalt sehr stoffwechselaktiver oder leistungsstarker Organe, wie z.B. dem Herzmuskel, zeigt. Es besteht ein ständiger Bedarf an neuen, nachweislich bioaktiven Komponenten mit Wirkung auf die biochemischen Prozesse, die zu schlechter Haut führen. Weiterhin besteht ein ständiger Bedarf an besonders hautverträglichen Formulierungen, die als Träger für Antitranspirant-Zusammensetzungen geeignet sind. Weiterhin besteht ein Bedarf an besonders hautverträglichen Formulierungen, die als Träger für Antitranspirant-Zusammensetzungen in der Lage sein können, die Haut mit wichtigen Nährstoffen zu versorgen und gegebenenfalls die die Haut belastende Wirkung des Antitranspirant-Wirkstoffs auszugleichen.

### Aufgabenstellung

Aufgabe der vorliegenden Erfindung war es, Wirkstoffe und/oder Träger für kosmetische und dermatologische Antitranspirant-Zusammensetzungen mit besonders hoher Hautverträglichkeit zu finden. Eine weitere Aufgabe der vorliegenden Erfindung war es, Wirkstoffe und/oder Träger für kosmetische und dermatologische Antitranspirant-Zusammensetzungen mit besonders hoher Hautverträglichkeit, insbesondere für mechanisch gereizte Haut, zu finden. Eine weitere Aufgabe der vorliegenden Erfindung war es, Wirkstoffe und/oder Träger für kosmetische und dermatologische Antitranspirant-Zusammensetzungen zu finden, die Antitranspirant-Wirkstoffe, die die Homöostase der Haut stören, hautverträglicher machen können. Eine weitere Aufgabe bestand darin, ein bioaktives Substanzgemisch zur Verfügung zu stellen, das die Synthese von Adenosintriphosphat (ATP) in den Hautzellen stimuliert. Die so bereitgestellte Energie steht der Haut für erhöhte Stoffwechselleistungen, z. B. die Hauterneuerung, oder Reparaturprozesse zur Verfügung. Letztlich wird so das Hautbild bei trockener oder geschädigter Haut von innen her verbessert durch Regulation eines relevanten biochemischen Prozesses. Ebenso wird die Schutzfunktion der Haut wiederhergestellt.

Überraschend wurde gefunden, dass eine Wirkstoffkombination aus Squalan, weiterhin mindestens einer Substanz, ausgewählt aus Tocopherol und Tocopherylestern sowie Mischungen hiervon, sowie mindestens einem Glycerindiester oder Glycerintriester von mindestens einer gesättigten, unsubstituierten Alkansäure mit 8 bis 18 Kohlenstoffatomen die gestellten Aufgaben löst und sehr gute pflegende und Haut nährende Eigenschaften besitzt sowie die Synthese von Adenosintriphosphat (ATP) in den Hautzellen stimuliert.

Gegenstand der vorliegenden Erfindung sind in einer ersten Ausführungsform kosmetische Antitranspirant-Zusammensetzungen, enthaltend
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,01 - 0,3 Gew.-% Squalan, weiterhin
c) insgesamt 0,01 - 0,5 Gew.-%, mindestens einer Substanz, ausgewählt aus Tocopherol und Tocopherylestern sowie Mischungen hiervon, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%,
wobei sich, sofern nichts anderes vermerkt ist, alle Gew.-%-Angaben auf das Gesamtgewicht der Antitranspirant-Zusammensetzung ohne Berücksichtigung ggf. vorhandener Treibmittel beziehen. Die Gew.-Angaben beziehen sich, sofern nichts anderes vermerkt ist, auf das Gesamtgewicht der Antitranspirant-Zusammensetzung, wobei ggf. vorhandene Treibmittel nicht berücksichtigt werden. Das heißt, dass sich die Mengenangaben für Antitranspirant-Sprays auf das Gesamtgewicht der treibmittelfreien Zusammensetzung (= Sud) beziehen.

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20 °C und ein Druck von 1013,25 hPa. Schmelzpunktangaben beziehen sich ebenfalls auf einen Druck von 1013,25 hPa.

"Freies Wasser" im Sinne der vorliegenden Anmeldung ist Wasser, das nicht in Form von Kristallwasser, Hydratationswasser oder ähnlich molekular gebundenem Wasser in der Antitranspirant-Zusammensetzung enthalten ist. Der Gehalt an Kristallwasser, Hydratationswasser oder ähnlich molekular gebundenem Wasser, der in den eingesetzten Bestandteilen, insbesondere in den schweißhemmenden Wirkstoffen, enthalten ist, stellt im Sinne der vorliegenden Anmeldung kein freies Wasser dar. Freies Wasser ist beispielsweise solches Wasser, das als Lösemittel, als Gelaktivator oder als Lösemittelbestandteil anderer Wirkstoffe zur erfindungsgemäßen Zusammensetzung zugegeben wird.

In einer ersten bevorzugten Ausführungsform haben die erfindungsgemäßen Antitranspirant-Zusammensetzungen einen Gehalt an freiem Wasser von 0 bis 6 Gew.-%, bevorzugt 1 - 5 Gew.-%, besonders bevorzugt 2 - 4 Gew.-%, jeweils bezogen auf das Gewicht der gesamten Antitranspirant-Zusammensetzung. Derartige Antitranspirant-Zusammensetzungen stellen bevorzugt Antitranspirant-Stifte, insbesondere Antitranspirant-Stifte auf wasserfreier Wachsbasis, oder niedrig-viskose (100 bis 15.000 mPas bei 20 °C) Flüssigkeiten dar, die mit Hilfe eines Treibmittels als Spray oder mit einer Rollkugel auf die Haut aufgetragen werden können.

In einer anderen bevorzugten Ausführungsform haben die erfindungsgemäßen Antitranspirant-Zusammensetzungen einen Gehalt an freiem Wasser von mehr als 6 bis 96 Gew.-%, bevorzugt 15 bis 85 Gew.-%, besonders bevorzugt 30 bis 75 Gew.-%, außerordentlich bevorzugt 50 bis 70 Gew.-%, jeweils bezogen auf das Gewicht der gesamten Antitranspirant-Zusammensetzung. Derartige Antitranspirant-Zusammensetzungen stellen bevorzugt Emulsionen dar. Besonders bevorzugte Antitranspirant-Zusammensetzungen dieser Art sind Öl-in-Wasser-Emulsionen, die mit einer Rollkugel auf die Haut aufgetragen werden können. Weitere bevorzugte Antitranspirant-Zusammensetzungen dieser Art sind Wasser-in-Öl-Emulsionen, die als mittel- bis hoch-viskoses (20.000 bis 100.000 mPas bei 20°C) Gel vorliegen.

### Antitranspirant-Wirkstoffe

Die Antitranspirant-Wirkstoffe sind ausgewählt aus den wasserlöslichen adstringierenden anorganischen und organischen Salzen des Aluminiums und von Aluminium-Zirkonium-Mischungen. Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 5 Gew.-% bei 20°C verstanden, das heißt, dass Mengen von wenigstens 5 g des Antitranspirant-Wirkstoffs in 95 g Wasser bei 20°C löslich sind.

Besonders bevorzugte Antitranspirant-Wirkstoffe sind ausgewählt aus Aluminiumchlorhydrat, insbesondere Aluminiumchlorhydrat mit der allgemeinen Formel [Al₂(OH)₅Cl · 1-6 H₂O]ₙ, bevorzugt [Al₂(OH)₅Cl · 2-3 H₂O]ₙ, das in nicht-aktivierter oder in aktivierter (depolymerisierter) Form vorliegen kann, sowie Aluminiumchlorhydrat mit der allgemeinen Formel [Al₂(OH)₄Cl_{2 ·} 1-6 H₂O]ₙ, bevorzugt [Al₂(OH)₄Cl₂ · 2-3 H₂O]ₙ, das in nicht-aktivierter oder in aktivierter (depolymerisierter) Form vorliegen kann.

Die Herstellung bevorzugter Antitranspirant-Wirkstoffe ist beispielsweise in US 3887692, US 3904741, US 4359456, GB 2048229 und GB 1347950 offenbart.

Weiterhin bevorzugt sind Aluminiumsesquichlorhydrat, Aluminiumdichlorhydrat, Aluminiumchlorohydrex-Propylenglycol (PG) oder Aluminiumchlorohydrex-Polyethylenglycol (PEG), Aluminium-Glycol-Komplexe, z. B. Aluminium-Propylenglycol-Komplexe, Aluminiumsesquichlorohydrex-PG oder Aluminiumsesquichlorohydrex-PEG, Aluminium-PG-dichlorohydrex oder Aluminium-PEGdichlorohydrex, Aluminiumhydroxid, weiterhin ausgewählt aus Kaliumaluminiumsulfat (KAl(SO₄)₂ · 12 H₂O, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Aluminiumsalzen von Lipoaminosäuren, Aluminiumsulfat, Aluminiumlactat, Aluminiumchlorhydroxyallantoinat und Natrium-Aluminium-Chlorhydroxylactat.

Erfindungsgemäß besonders bevorzugte Antitranspirant-Wirkstoffe sind ausgewählt aus so genannten "aktivierten" Aluminiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Ihre Herstellung ist beispielsweise in GB 2048229, US 4775528 und US 6010688 offenbart. Aktivierte Aluminiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminiumsalzes, beispielsweise gemäß US 6010688, die durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt Propylenglycol, Butylenglycol, Diethylenglycol, Dipropylenglycol, Glycerin, Sorbit und Pentaerythrit, besonders bevorzugt Propylenglycol.

Besonders bevorzugt sind auch Komplexe aktivierter schweißhemmender Aluminiumsalze mit einem mehrwertigen Alkohol, die 20 - 50 Gew.-%, besonders bevorzugt 20 - 42 Gew.-%, aktiviertes schweißhemmendes Aluminiumsalz und 2 - 16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole. Derartige erfindungsgemäß bevorzugte Komplexe eines aktivierten schweißhemmenden Aluminiumsalzes mit einem mehrwertigen Alkohol sind z. B. offenbart in US 5643558 und US 6245325.

Weitere bevorzugte schweißhemmende Wirkstoffe sind basische Calcium-Aluminiumsalze, wie sie beispielsweise in US 2571030 offenbart sind. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind aktivierte Aluminiumsalze, wie sie beispielsweise in US 6245325 oder US 6042816 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Aluminium - Gewichtsverhältnis von 2:1 - 1:20 und bevorzugt 1:1 bis 1:10 bereitzustellen, sowie ein wasserlösliches Calciumsalz in einer solchen Menge, um ein Ca:Al-Gewichtsverhältnis von 1:1 - 1:28 und bevorzugt 1:2 - 1:25 bereitzustellen.

Besonders bevorzugte feste aktivierte schweißhemmende Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Aminosäure, dass das Aminosäure zu (AI+Zr) - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser (Hydratationswasser), weiterhin soviel wasserlösliches Calciumsalz, dass das Ca:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6245325 oder US 6042816, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Calciumsalz, dass das Ca:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Für die Stabilisierung der schweißhemmenden Salze bevorzugte wasserlösliche Calciumsalze sind ausgewählt aus Calciumchlorid, Calciumbromid, Calciumnitrat, Calciumcitrat, Calciumformiat, Calciumacetat, Calciumgluconat, Calciumascorbat, Calciumlactat, Calciumglycinat, Calciumcarbonat, Calciumsulfat, Calciumhydroxid, sowie Mischungen davon.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und y-Amino-n-butansäure und den Salzen davon, jeweils in der d-Form, der l-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte schweißhemmende Wirkstoffe sind aktivierte Aluminiumsalze, wie sie beispielsweise in US 6902723 offenbart sind, enthaltend 5 - 78 Gew.-% (USP) eines aktivierten schweißhemmenden Aluminiumsalzes, eine Aminosäure oder Hydroxyalkansäure in einer solchen Menge, um ein (Aminosäure oder Hydroxyalkansäure) zu Al - Gewichtsverhältnis von 2:1 - 1:20 und bevorzugt 1:1 bis 1:10 bereitzustellen, sowie ein wasserlösliches Strontiumsalz in einer solchen Menge, um ein Sr:Al-Gewichtsverhältnis von 1:1 - 1:28 und bevorzugt 1:2 - 1:25 bereitzustellen.

Besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Aminosäure, dass das Aminosäure zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Glycin, dass das Glycin zu Al - Gewichtsverhältnis 2:1 - 1:20, bevorzugt 1:1 - 1:10, beträgt.

Weitere besonders bevorzugte feste schweißhemmende aktivierte Salzzusammensetzungen, beispielsweise gemäß US 6902723, enthalten 48 - 78 Gew.-% (USP), bevorzugt 66 - 75 Gew.-% eines aktivierten Aluminiumsalzes und 1 - 16 Gew.-%, bevorzugt 4 - 13 Gew.-% molekular gebundenes Wasser, weiterhin soviel wasserlösliches Strontiumsalz, dass das Sr:Al-Gewichtsverhältnis 1:1 - 1:28, bevorzugt 1:2 - 1:25, beträgt und soviel Hydroxyalkansäure, dass das Hydroxyalkansäure zu Al - Gewichtsverhältnis 2:1 - 1:20, bevorzugt 1:1 - 1:10, beträgt.

Weitere bevorzugte aktivierte Aluminiumsalze sind solche der allgemeinen Formel Al₂(OH)₆₋ₐXa, worin X Cl, Br, I oder NO₃ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt, wie sie beispielsweise in US 6074632 offenbart sind. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Weitere bevorzugte schweißhemmende Wirkstoffe sind in US 6663854 und US 20040009133 offenbart.

Die schweißhemmenden Wirkstoffe können sowohl in gelöster oder solubilisierter Form auch als auch in ungelöster, suspendierter Form vorliegen. Die Antitranspirant-Wirkstoffe können pulverförmig, als wässrige Lösung, nicht-wässrige Lösungen oder als glycolische Solubilisate eingesetzt werden.

Sofern die schweißhemmenden Wirkstoffe in einem mit Wasser nicht mischbaren Träger suspendiert vorliegen, ist es aus Gründen der Produktstabilität bevorzugt, dass die Wirkstoffpartikel eine zahlenmittlere Partikelgröße von 0,1 - 200 µm, bevorzugt 1 - 50 µm, besonders bevorzugt 3 - 20 µm und außerordentlich bevorzugt 5 - 10 µm, aufweisen. Bevorzugte Wirkstoffpartikel weisen eine volumenmittlere Partikelgröße von 0,2 - 220 µm, bevorzugt 3 - 60 µm, besonders bevorzugt 4 - 25 µm, weiterhin bevorzugt 5 - 20 µm und außerordentlich bevorzugt 10 - 15,5 µm, auf. Bevorzugte Aluminiumsalze weisen ein molares Metall-zu-Chlorid-Verhältnis von 1,9 -2,1, bzw. für Sesquichlorohydrate von 1,5:1-1,8:1 auf.

Besonders bevorzugte erfindungsgemäße Zusammensetzungen sind dadurch gekennzeichnet, dass der mindestens eine Antitranspirant-Wirkstoff in einer Menge von 3 - 30 Gew.-%, bevorzugt 10 - 28 Gew.-%, besonders bev Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf.

Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf. orzugt 15 - 27 Gew.-% und außerordentlich bevorzugt 23-26 Gew.-%, enthalten ist, bezogen auf das Gesamtgewicht der kristallwasserfreien Aktivsubstanz (USP) in der treibmittelfreien Gesamtzusammensetzung.

In einer besonders bevorzugten Ausführungsform enthält die Zusammensetzung ein adstringierendes Aluminiumsalz, insbesondere Aluminiumchlorohydrat, besonders bevorzugt Aluminiumchlorohydrat mit einer kristallwasserfreien Aktivsubstanz (USP) von 72 - 88 Gew.-%, bezogen auf den Rohstoff tel quel. Bevorzugte nicht-aktivierte Aluminiumchlorohydrate sind beispielsweise pulverförmig als Micro Dry^{®}, Micro Dry^{®} Ultrafine oder Micro Dry^{®}-323 von Summit, als Chlorhydrol^{®} (Pulver) sowie in aktivierter Form als Reach^{®} 101, Reach^{®} 103, Reach^{®} 501 von Reheis/Summit oder AACH-7171 von Summit vertrieben wird. Unter der Bezeichnung Reach^{®} 301 wird ein Aluminiumsesquichlorohydrat von Reheis angeboten, das ebenfalls besonders bevorzugt ist. Besonders bevorzugt sind auch aktivierte Aluminiumchlorohydrate, die unter den Bezeichnungen Reach^{®} 101 und Reach^{®} 103, AACH- 7171 von Reheis oder Summit erhältlich sind. Auch die Verwendung von Aluminium-Zirkonium-Tetrachlorohydrex-Glycin-Komplexen, die beispielsweise von Reheis unter der Bezeichnung Rezal^{®} 36 GP von Reheis oder AZG-364 oder 369 von Summit, in aktivierter Qualität, als Reach^{®} 908, als Pulver im Handel sind, kann erfindungsgemäß besonders bevorzugt sein. Auch Aluminium-Zirkoniumpentachlorohydex-Glycin-Komplexe (AAZG-3108 oder AAZG-3110 von Summit) sind bevorzugte Antitranspirant-Wirkstoffe.

Ein erster obligatorischer Bestandteil der erfindungsgemäßen Wirkstoffkombination zur Steigerung der ATP-Synthese in den Hautzellen ist Squalan, das in den erfindungsgemäßen Antitranspirant-Zusammensetzungen insgesamt in einer Menge von 0,01 - 0,3 Gew.-%, bevorzugt 0,05 - 0,2 Gew.-%, besonders bevorzugt 0,1 - 0,15 Gew.-%, jeweils bezogen auf das Gewicht der gesamten erfindungsgemäßen Antitranspirant-Zusammensetzung, enthalten ist.

Ein zweiter obligatorischer Bestandteil der erfindungsgemäßen Wirkstoffkombination zur Steigerung der ATP-Synthese in den Hautzellen ist eine Substanz, die ausgewählt ist aus Tocopherolen und Tocopherylestern sowie Mischungen hiervon, die in den erfindungsgemäßen Antitranspirant-Zusammensetzungen insgesamt in einer Menge von 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-%, jeweils bezogen auf das Gewicht der gesamten erfindungsgemäßen Antitranspirant-Zusammensetzung, enthalten ist. Ein bevorzugtes Tocopherol ist alpha-Tocopherol. Bevorzugte Tocopherylester sind die Ester des alpha-Tocopherols, insbesondere alpha-Tocopherylacetat, alpha-Tocopherylpalmitat, alpha-Tocopherylphosphat, alpha-Tocopherylsuccinat, alpha-Tocopherylinoleat und alpha-Tocopheryloleat. Außerordentlich bevorzugt ist alpha-Tocopherylacetat. Weiterhin bevorzugt sind Mischungen aus alpha-Tocopherol und alpha-Tocopherylacetat. Besonders bevorzugt sind die racemischen Gemische dieser Substanzen. Erfindungsgemäß bevorzugte Antitranspirant-Zusammensetzungen enthalten 0,01 - 0,3 Gew.-%, bevorzugt 0,05 - 0,2 Gew.-%, besonders bevorzugt 0,1 - 0,15 Gew.-%, Squalan und 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat, jeweils bezogen auf das Gewicht der gesamten erfindungsgemäßen Antitranspirant-Zusammensetzung.

Ein dritter obligatorischer Bestandteil der erfindungsgemäßen Wirkstoffkombination zur Steigerung der ATP-Synthese in den Hautzellen ist eine Substanz, die ausgewählt ist aus Glycerindiestern und Glycerintriestern von mindestens einer gesättigten, unsubstituierten Alkansäure mit 8 bis 18 Kohlenstoffatomen in einer Gesamtmenge von 0,1 - 5 Gew.-%, bezogen auf das Gewicht der gesamten erfindungsgemäßen Antitranspirant-Zusammensetzung. Bevorzugte Glycerindiester sind Glycerindi-n-octanoat, Glycerindi-n-decanoat, Glycerindilaurat, Glycerindi-n-tetradecanoat, Glycerindipalmitat und Glycerindistearat sowie Mischungen hiervon. Bevorzugte Glycerintriester sind Glycerintri-n-octanoat, Glycerintri-n-decanoat, Glycerintrilaurat, Glycerintri-n-tetradecanoat, Glycerintripalmitat und Glycerintristearat sowie Mischungen hiervon. Weiterhin bevorzugt sind Mischungen, enthaltend Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat. Besonders bevorzugt sind Mischungen, enthaltend Glycerindi-n-octanoat, Glycerindi-n-decanoat, Glycerindilaurat, Glycerindi-n-tetradecanoat, Glycerindistearat, Glycerintri-n-octanoat, Glycerintri-n-decanoat, Glycerintrilaurat, Glycerintri-n-tetradecanoat und Glycerintristearat. Der genannte oder die genannten Glycerindiester und/oder Glycerintriester sind in einer Gesamtmenge von 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, enthalten, jeweils bezogen auf das Gewicht der gesamten erfindungsgemäßen Antitranspirant-Zusammensetzung. Bevorzugte Antitranspirant-Zusammensetzungen enthalten, jeweils bezogen auf ihr Gewicht, insgesamt 0,2 bis 4,5 Gew.-%, besonders bevorzugt 0,3 bis 4 Gew.-%, außerordentlich bevorzugt 0,5 bis 3,5 Gew.-%, mindestens einer Substanz, ausgewählt aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat sowie Mischungen dieser Substanzen. Besonders bevorzugte Antitranspirant-Zusammensetzungen enthalten, jeweils bezogen auf ihr Gewicht, insgesamt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat. Außerordentlich bevorzugte Antitranspirant-Zusammensetzungen enthalten, jeweils bezogen auf ihr Gewicht, insgesamt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindistearat, Glycerintri-n-octanoat, Glycerintri-n-decanoat, Glycerintrilaurat, Glycerintri-n-tetradecanoat und Glycerintristearat.

Weitere erfindungsgemäß bevorzugte Antitranspirant-Zusammensetzungen enthalten, jeweils bezogen auf ihr Gesamtgewicht, 0,01 - 0,3 Gew.-%, bevorzugt 0,05 - 0,2 Gew.-%, besonders bevorzugt 0,1 - 0,15 Gew.-%, Squalan; zusätzlich dazu 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat; und zusätzlich dazu insgesamt 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, mindestens einer Substanz, die ausgewählt ist aus Glycerindiestern und Glycerintriestern von mindestens einer gesättigten, unsubstituierten Alkansäure mit 8 bis 18 Kohlenstoffatomen. Weitere erfindungsgemäß besonders bevorzugte Antitranspirant-Zusammensetzungen enthalten, jeweils bezogen auf ihr Gesamtgewicht, 0,01 - 0,3 Gew.-%, bevorzugt 0,05 - 0,2 Gew.-%, besonders bevorzugt 0,1 - 0,15 Gew.-%, Squalan; zusätzlich dazu 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat; und zusätzlich dazu insgesamt 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat. Weitere erfindungsgemäß besonders bevorzugte Antitranspirant-Zusammensetzungen enthalten, jeweils bezogen auf ihr Gesamtgewicht, 0,01 - 0,3 Gew.-%, bevorzugt 0,05 - 0,2 Gew.-%, besonders bevorzugt 0,1 - 0,15 Gew.-%, Squalan; zusätzlich dazu 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat; und zusätzlich dazu insgesamt 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindistearat, Glycerintri-n-octanoat, Glycerintri-n-decanoat, Glycerintrilaurat, Glycerintri-n-tetradecanoat und Glycerintristearat.

In den nachfolgenden Tabellen sind erfindungsgemäß bevorzugte und besonders bevorzugte Antitranspirant-Stiftzusammensetzungen Nr. 1 bis Nr. 20 zusammengestellt. Alle Mengenangaben sind in Gew.-%, bezogen auf das Gesamtgewicht der Stiftzusammensetzung.

| | Nr. 1 | Nr. 2 | Nr. 3 | Nr. 4 |
|---|---|---|---|---|
| Antitranspirant-Wirkstoff(e) insgesamt, ausgewählt aus Aluminium-Salzen und Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens einen Glycerindiester oder Glycerintriester von mindestens einer gesättigten, unsubstituierten Alkansäure mit 8 bis 18 Kohlenstoffatomen in einer Gesamtmenge von | 0,1 - 5 | 0,2 - 4,5 | 0,3 - 4 | 0,5 - 3,5 |

| | Nr. 5 | Nr. 6 | Nr. 7 | Nr. 8 |
|---|---|---|---|---|
| Antitranspirant-Wirkstoff(e) insgesamt, ausgewählt aus Aluminium-Salzen und Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens 1 Substanz, ausgewählt aus Glycerindi-n-octanoat, Glycerindi-n-decanoat, Glycerindilaurat, Glycerindi-n-tetradeca-noat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat u. Mischungen | 0,1 - 5 | 0,2 - 4,5 | 0,3 - 4 | 0,5 - 3,5 |

| | Nr. 9 | Nr. 10 | Nr. 11 | Nr. 12 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindipalmitat, Glycerindistearat, Glycerintri-n-octanoat, Glycerintri-n-decanoat, Glycerintrilaurat, Glycerintri-n-tetradecanoat, Glycerintripalmitat u. Gly-cerintristearat | 0,1 - 5 | 0,2 - 4,5 | 0,3 - 4 | 0,5 - 3,5 |

| | Nr. 13 | Nr. 14 | Nr. 15 | Nr. 16 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindistearat, Glycerintri-n-octanoat, Glyce-rintri-n-decanoat, Glycerintrilaurat, Glycerintri-n-tetra-decanoat und Glycerintristearat | 0,1 - 5 | 0,2 - 4,5 | 0,3 - 4 | 0,5 - 3,5 |

| | Nr. 17 | Nr. 18 | Nr. 19 | Nr. 20 |
|---|---|---|---|---|
| Antitranspirant-Wrkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| Tocopherylacetat | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindistearat, Glycerintri-n-octanoat, Glyce-rintri-n-decanoat, Glycerintrilaurat, Glycerintri-n-tetra-decanoat und Glycerintristearat | 0,1 - 5 | 0,2 - 4,5 | 0,3 - 4 | 0,5 - 3,5 |

### Zusatzstoffe für wasserfreie Darreichungsformen

Erfindungsgemäße Antitranspirant-Zusammensetzungen mit einem Gehalt an freiem Wasser von 0 - 6 Gew.-%, bezogen auf das Gesamtgewicht der Zusammensetzung, sind dadurch gekennzeichnet, dass, bezogen auf das Gewicht der gesamten Zusammensetzung, insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen enthalten sind. Überraschend wurde festgestellt, dass durch diesen Zusatzstoff die ATP-Synthese in den Hautzellen weiter angeregt werden konnte. Bevorzugte gesättigte, unsubstituierte Alkansäuren mit 12 bis 18 Kohlenstoffatomen sind ausgewählt aus n-Dodecansäure, n-Tridecansäure, n-Tetradecansäure, n-Pentadecansäure, n-Hexadecansäure (Palmitinsäure), n-Heptadecansäure und n-Octadecansäure (Stearinsäure) sowie Mischungen hiervon. Besonders bevorzugt sind n-Dodecansäure, n-Tetradecansäure, n-Hexadecansäure (Palmitinsäure) und n-Octadecansäure (Stearinsäure) sowie Mischungen hiervon. Außerordentlich bevorzugt sind n-Hexadecansäure (Palmitinsäure) und n-Octadecansäure (Stearinsäure) sowie Mischungen hiervon, insbesondere eine Mischung aus n-Hexadecansäure (Palmitinsäure) und n-Octadecansäure (Stearinsäure) im Molverhältnis 1:1.

Bevorzugte Antitranspirant-Zusammensetzungen enthalten, jeweils bezogen auf das Gesamtgewicht der Zusammensetzung, 0 - 6 Gew.-%, bevorzugt 1 - 5 Gew.-%, besonders bevorzugt 2 - 4 Gew.-%, freies Wasser und insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, ausgewählt aus n-Dodecansäure, n-Tetradecansäure, n-Hexadecansäure (Palmitinsäure) und n-Octadecansäure (Stearinsäure) sowie Mischungen hiervon.

Weitere bevorzugte Antitranspirant-Zusammensetzungen enthalten, jeweils bezogen auf das Gesamtgewicht der Zusammensetzung, 0 - 6 Gew.-%, bevorzugt 1 - 5 Gew.-%, besonders bevorzugt 2 - 4 Gew.-%, freies Wasser und insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, ausgewählt aus n-Hexadecansäure (Palmitinsäure) und n-Octadecansäure (Stearinsäure) sowie Mischungen hiervon, insbesondere ausgewählt aus einer Mischung aus n-Hexadecansäure (Palmitinsäure) und n-Octadecansäure (Stearinsäure) im Molverhältnis 1:1.

In den nachfolgenden Tabellen sind erfindungsgemäß bevorzugte und besonders bevorzugte Antitranspirant-Stiftzusammensetzungen Nr. 21 bis Nr. 36 zusammengestellt. Alle Mengenangaben sind in Gew.-%, bezogen auf das Gesamtgewicht der Stiftzusammensetzung.

| | Nr. 21 | N r. 22 | Nr. 23 | Nr.24 |
|---|---|---|---|---|
| Antitranspirant-Wirkstoff(e) insgesamt, ausgewählt aus Aluminium-Salzen und Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens einen Glycerindiester oder Glycerintriester von mindestens einer gesättigten, unsubstituierten Alkansäure mit 8 bis 18 Kohlenstoffatomen in einer Gesamtmenge von | 0,1 - 5 | 0,2 - 4,5 | 0,3 - 4 | 0,5 - 3,5 |
| freies Wasser | 0 - 6 | 1 - 5 | 2 - 4 | 2 - 4 |
| insgesamt mind. 1 gesättigte, unsubstituierte Alkansäure mit 12 - 18 Kohlenstoffatomen | 0,05 - 0,5 | 0,1 - 0,4 | 0,2 - 0,3 | 0,2 - 0,3 |

| | Nr. 25 | Nr. 26 | Nr. 27 | Nr. 28 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens 1 Substanz, ausgewählt aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradeca-noat, Glycerintripalmitat und Glycerintristearat u. | 0,1 - 5 | 0,2 - 4,5 | 0,3 - 4 | 0,5 - 3,5 |
| Mischungen | | | | |
| freies Wasser | 0 - 6 | 1 - 5 | 2 - 4 | 2 - 4 |
| insgesamt mind. 1 gesättigte, unsubst. Alkansäure mit 12 - 18 C-Atomen, ausgewählt aus n-Dodecan-säure, n-Tridecansäure, n-Tetradecansäure, n-Penta-decansäure, n-Hexadecansäure , n-Heptadecansäure und n-Octadecansäure sowie Mischungen hiervon | 0,05 - 0,5 | 0,1 - 0,4 | 0,2 - 0,3 | 0,2 - 0,3 |

| | Nr. 29 | Nr. 30 | Nr. 31 | Nr. 32 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindipalmitat, Glycerindistearat, Glyce-rintri-n-octanoat, Glycerintri-n-decanoat, Glycerin-trilaurat, Glycerintri-n-tetradecanoat, Glycerintripal-mitat u. Glycerintristearat | 0,1 - 5 | 0,2 - 4,5 | 0,3 - 4 | 0,5 - 3,5 |
| freies Wasser | 0 - 6 | 1 - 5 | 2 - 4 | 2 - 4 |
| insgesamt mind. 1 gesättigte, unsubst. Alkansäure mit 12 - 18 C-Atomen, ausgewählt aus n-Dodecan-säure, n-Tetradecansäure, n-Hexadecansäure und n-Octadecansäure sowie Mischungen hiervon | 0,05 - 0,5 | 0,1 - 0,4 | 0,2 - 0,3 | 0,2 - 0,3 |

| | Nr. 33 | Nr. 34 | Nr. 35 | Nr. 36 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindistearat, Glycerintri-n-octanoat, Glycerintri-n-decanoat, Glycerintrilaurat, Glycerintri-n-tetradecanoat und Glycerintristearat | 0,1 - 5 | 0,2 - 4,5 | 0,3 - 4 | 0,5 - 3,5 |
| freies Wasser | 0 - 6 | 1 - 5 | 2 - 4 | 2 - 4 |
| insgesamt mind. 1 gesättigte, unsubst. Alkansäure mit 12 - 18 C-Atomen, ausgewählt aus Mischung aus n-Hexadecansäure und n-Octadecansäure im Molverhältnis 1:1 | 0,05 - 0,5 | 0,1 - 0,4 | 0,2 - 0,3 | 0,2 - 0,3 |

### Zusatzstoffe für wasserhaltige Darreichungsformen

Erfindungsgemäße Antitranspirant-Zusammensetzungen mit einem Gehalt an freiem Wasser von mehr als 6 bis 96 Gew.-%, bevorzugt 15 bis 85 Gew.-%, besonders bevorzugt 30 bis 75 Gew.-%, außerordentlich bevorzugt 50 bis 70 Gew.-%, Gew.-%, jeweils bezogen auf das Gesamtgewicht der Zusammensetzung, sind dadurch gekennzeichnet, dass, bezogen auf das Gewicht der gesamten Zusammensetzung, Glycerin in einer Gesamtmenge von 0,01 bis 0,5 Gew.-%, bevorzugt 0,05 bis 0,3 Gew.-%, besonders bevorzugt 0,1 bis 0,2 Gew.-%, enthalten ist. Überraschend wurde festgestellt, dass durch diesen Zusatzstoff die ATP-Synthese in den Hautzellen weiter angeregt werden konnte.

Weitere erfindungsgemäße Antitranspirant-Zusammensetzungen mit einem Gehalt an freiem Wasser von mehr als 6 bis 96 Gew.-%, bevorzugt 15 bis 85 Gew.-%, besonders bevorzugt 30 bis 75 Gew.-%, außerordentlich bevorzugt 50 bis 70 Gew.-%, Gew.-%, jeweils bezogen auf das Gesamtgewicht der Zusammensetzung, sind dadurch gekennzeichnet, dass, bezogen auf das Gewicht der gesamten Zusammensetzung, Ca²⁺ in einer Gesamtmenge von 0,001 bis 0,1 Gew.-%, bevorzugt 0,01 bis 0,08 Gew.-%, besonders bevorzugt 0,03 bis 0,06 Gew.-%, außerordentlich bevorzugt 0,04 bis 0,05 Gew.-%, enthalten ist. Überraschend wurde festgestellt, dass durch diesen Zusatzstoff die ATP-Synthese in den Hautzellen weiter angeregt werden konnte. Ca²⁺ wird in Form eines wasserlöslichen Salzes zugesetzt, wobei sowohl anorganische als auch organische Ca²⁺-Salze geeignet sind, sofern sie wasserlöslich sind. Bevorzugt ist Calciumchlorid, CaCl₂. Erfindungsgemäß wird unter Wasserlöslichkeit eine Löslichkeit von wenigstens 5 Gew.-% bei 20 °C verstanden, das heißt, dass Mengen von wenigstens 5 g des Ca²⁺-Salzes in 95 g Wasser bei 20 °C löslich sind.

Weitere erfindungsgemäße Antitranspirant-Zusammensetzungen mit einem Gehalt an freiem Wasser von mehr als 6 bis 96 Gew.-%, bevorzugt 15 bis 85 Gew.-%, besonders bevorzugt 30 bis 75 Gew.-%, außerordentlich bevorzugt 50 bis 70 Gew.-%, Gew.-%, jeweils bezogen auf das Gesamtgewicht der Zusammensetzung, sind dadurch gekennzeichnet, dass, bezogen auf das Gewicht der gesamten Zusammensetzung, Ca²⁺ in einer Gesamtmenge von 0,001 bis 0,1 Gew.-%, bevorzugt 0,01 bis 0,08 Gew.-%, besonders bevorzugt 0,03 bis 0,06 Gew.-%, außerordentlich bevorzugt 0,04 bis 0,05 Gew.-%, und weiterhin Glycerin in einer Gesamtmenge von 0,01 bis 0,5 Gew.-%, bevorzugt 0,05 bis 0,3 Gew.-%, besonders bevorzugt 0,1 bis 0,2 Gew.-%, enthalten sind. Besonders bevorzugte Antitranspirant-Zusammensetzungen enthalten, jeweils bezogen auf ihr Gesamtgewicht, 30 bis 75 Gew.-% freies Wasser, 0,03 bis 0,06 Gew.-% Ca²⁺ und weiterhin 0,05 bis 0,3 Gew.-% Glycerin.

In den nachfolgenden Tabellen sind erfindungsgemäß bevorzugte und besonders bevorzugte Antitranspirant-Stiftzusammensetzungen Nr. 37 bis Nr. 52 zusammengestellt. Alle Mengenangaben sind in Gew.-%, bezogen auf das Gesamtgewicht der Stiftzusammensetzung.

| | Nr. 37 | Nr. 38 | Nr. 39 | Nr. 40 |
|---|---|---|---|---|
| Antitranspirant-Wirkstoff(e) insgesamt, ausgewählt aus Aluminium-Salzen und Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens einen Glycerindiester oder Glycerintriester von mindestens einer gesättigten, unsubstituierten Alkansäure mit 8 bis 18 Kohlenstoffatomen in einer Gesamtmenge von | 0,1 - 5 | 0,2 - 4,5 | 0,3 - 4 | 0,5 - 3,5 |
| freies Wasser | 6 - 96 | 15 - 85 | 30 - 75 | 50 - 70 |
| Ca²⁺ | 0,001 - 0,1 | 0,01 - 0,08 | 0,03 - 0,06 | 0,04 - 0,05 |

| | Nr. 41 | Nr. 42 | Nr. 43 | Nr. 44 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen hiervon | 0,01 - 0,5 | 0,1 - 0,4 | 0,2 - 0,35 | 0,25 - 0,3 |
| mindestens einen Glycerindiester oder Glycerintriester von mindestens einer gesättigten, unsubstituierten Alkansäure mit 8 bis 18 Kohlenstoffatomen in einer Gesamtmenge von | 0,1 - 5 | 0,2 - 4,5 | 0,3 - 4 | 0,5 - 3,5 |
| freies Wasser | 6 - 96 | 15 - 85 | 30 - 75 | 50 - 70 |
| Ca²⁺ | 0,001 - 0,1 | 0,01 - 0,08 | 0,03 - 0,06 | 0,04 - 0,05 |
| Glycerin | 0,01 - 0,5 | 0,05 - 0,3 | 0,1 - 0,2 | 0,1 - 0,2 |

| | Nr. 45 | N r. 46 | Nr. 47 | Nr. 48 |
|---|---|---|---|---|
| Antitranspirant-Wrkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindipalmitat, Glycerindistearat, Glyce-rintri-n-octanoat, Glycerintri-n-decanoat, Glycerin-trilaurat, Glycerintri-n-tetradecanoat, Glycerintripal-mitat u. Glycerintristearat | 0,1 - 5 | 0,2 - 4,5 | 0,3 - 4 | 0,5 - 3,5 |
| freies Wasser | 6 - 96 | 15 - 85 | 30 - 75 | 50 - 70 |
| Ca²⁺ | 0,001 - 0,1 | 0,01 - 0,08 | 0,03 - 0,06 | 0,04 - 0,05 |
| Glycerin | 0,01 - 0,5 | 0,05 - 0,3 | 0,1 - 0,2 | 0,1 - 0,2 |

| | Nr. 49 | Nr. 50 | Nr. 51 | Nr. 52 |
|---|---|---|---|---|
| Antitranspirant-Wirkstoff(e) insgesamt, ausgewählt aus Aluminium-Salzen u Aluminium-Zirkonium-Salzen | 3 - 30 | 10 - 28 | 15 - 27 | 23 - 26 |
| Squalan | 0,01 - 0,3 | 0,05 - 0,2 | 0,1 - 0,15 | 0,1 - 0,15 |
| insgesamt mindestens 1 Substanz, ausgewählt aus Tocopherolen und Tocopherylestern u. Mischungen | 0,01 - 0,5 | 0,1 - 0,4 | 0,2 - 0,35 | 0,25 - 0,3 |
| Mischung aus Glycerindi-n-octanoat, Glycerindi-n-decanoat, Glycerindilaurat, Glycerindi-n-tetradeca-noat, Glycerindistearat, Glycerintri-n-octanoat, Glycerintri-n-decanoat, Glycerintrilaurat, Glycerintri-n-tetradecanoat und Glycerintristearat | 0,1 - 5 | 0,2 - 4,5 | 0,3 - 4 | 0,5 - 3,5 |
| freies Wasser | 6 - 96 | 15 - 85 | 30 - 75 | 50 - 70 |
| Ca²⁺ | 0,001 - 0,1 | 0,01 - 0,08 | 0,03 - 0,06 | 0,04 - 0,05 |
| Glycerin | 0,01 - 0,5 | 0,05 - 0,3 | 0,1 - 0,2 | 0,1 - 0,2 |

Die erfindungsgemäßen Antitranspirant-Zusammensetzungen mit einem Gehalt an freiem Wasser von 0 - 6 Gew.-%, bezogen auf das Gesamtgewicht der Zusammensetzung, enthalten mindestens ein kosmetisches Öl als Träger.

Antitranspirant-Stifte mit einem Gehalt an freiem Wasser von 0 - 6 Gew.-%, bezogen auf das Gesamtgewicht der Zusammensetzung, können in gelierter Form vorliegen, wobei die Ölphase mindestens eine Siliconkomponente enthalten oder aus mindestens einer Siliconkomponente bestehen kann. Weiterhin können die erfindungsgemäßen Zusammensetzungen, die als Antitranspirant-Stifte formuliert sind, auf wasserfreier Fettbasis, auf Basis einer Polyol-in-Öl-Emulsion, auf Basis einer Öl-in-Polyol-Emulsion, auf Basis einer Polyol-Öl-Mehrfach-Emulsion, auf Basis einer Nanoemulsion und auf Basis einer Mikroemulsion vorliegen, wobei die Polyolphase wasserfrei sein oder nur einen geringen Wassergehalt (z. B. 0,5 - 3 Gew.-%, bezogen auf die gesamte Zusammensetzung) aufweisen kann. Gelstifte können auf der Basis von Alditolen, insbesondere Dibenzylidensorbitol, N-Acylaminosäureamiden, 12-Hydroxystearinsäure, Polyamiden und Polyamidderivaten, formuliert werden.

Antitranspirant-Wachsstifte mit einem Gehalt an freiem Wasser von 0 - 6 Gew.-%, bezogen auf das Gesamtgewicht der Zusammensetzung, enthalten etwa 30 - 70 Gew.-% an mindestens einem unter Normalbedingungen flüssigen kosmetischen Öl, etwa 15 - 25 Gew.-% einer unter Normalbedingungen festen Fettkomponente, wovon üblicherweise der größte Anteil einen Schmelzpunkt von ca. 50 °C aufweist bzw. üblicherweise aus Fettalkoholen, insbesondere aus Stearylalkohol, aber auch Cetylalkohol und ggf. noch Arachidylalkohol und/oder Behenylalkohol besteht, während ein geringerer Anteil - etwa 0,5 - 5 Gew.-% - aus mindestens einer Fettkomponente mit einem Schmelzpunkt von ca. 55 - 120 °C besteht. Weiterhin können 0,5 - 30 Gew.-% mindestens eines Füllstoffes enthalten sein, der typischerweise ausgewählt ist aus Talkum, Cellulosepulvern, Stärken und Stärkederivaten. Weiterhin können 0,1 - 10 Gew.-%, bevorzugt 1 - 5 Gew.-%, besonders bevorzugt 2 - 4 Gew.-% mindestens eines Öl-in-Wasser-Emulgators enthalten sein.

Eine erfindungsgemäß bevorzugte Ausführungsform als Antitranspirant-Wachsstift mit einem Gehalt an freiem Wasser von 0 - 6 Gew.-%, bezogen auf das Gesamtgewicht der Zusammensetzung, ist dadurch gekennzeichnet, dass enthalten sind:
insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
insgesamt 0,01 - 0,3 Gew.-% Squalan, weiterhin
insgesamt 0,01 - 0,5 Gew.-%, mindestens einer Substanz, ausgewählt aus Tocopherolen und
Tocopherylestern sowie Mischungen hiervon,
mindestens einen Glycerindiester oder Glycerintriester von mindestens einer gesättigten,
unsubstituierten Alkansäure mit 8 bis 18 Kohlenstoffatomen in einer Gesamtmenge von 0,1 - 5 Gew.-%,
optional insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen,
30 - 70 Gew.-% an mindestens einem unter Normalbedingungen flüssigen kosmetischen Öl, 15 - 32 Gew.-% einer unter Normalbedingungen festen Fettkomponente, wovon mehr als 65 Gew.-%, bevorzugt mehr als 70 Gew.-%, besonders bevorzugt mehr als 80 Gew.-%, jeweils bezogen auf den Gesamtgehalt an unter Normalbedingungen festen Fettkomponenten, aus C₁₆-C₃₀-Fettalkoholen, bevorzugt ausgewählt aus der Gruppe, bestehend aus Stearylalkohol, Cetylalkohol und Mischungen hiervon, besteht, wobei besonders bevorzugt zusätzlich zu Stearylalkohol und/oder Cetylalkohol weiterhin 0,1 - 3 Gew.-%, bezogen auf den gesamten Stift, Arachidylalkohol und/oder Behenylalkohol und/oder mindestens ein C₂₄-C₃₀-Fettalkohol enthalten ist/sind,
weiterhin 0,5 - 5 Gew.-%, bezogen auf den gesamten Stift, mindestens einer Fettkomponente mit einem Schmelzpunkt von 75 - 120 °C,
weiterhin 0,5 - 30 Gew.-%, bevorzugt 1 - 25 Gew.-%, besonders bevorzugt 5 - 20 Gew.-%, außerordentlich bevorzugt 10 - 15 Gew.-%, mindestens eines Füllstoffes, der besonders bevorzugt ausgewählt ist aus Talkum, Cellulosepulvern, Stärken und Stärkederivaten.

Erfindungsgemäß bevorzugte Antitranspirant-Zusammensetzungen mit einem Gehalt an freiem Wasser von 0 - 6 Gew.-%, bezogen auf das Gesamtgewicht der Zusammensetzung, und zwar sowohl Stifte als auch Spray-Suds oder Roll-on-Zusammensetzungen, enthalten 30 - 70 Gew.-%, bezogen auf die gesamte Zusammensetzung, an mindestens einem unter Normalbedingungen flüssigen kosmetischen Öl.

Erfindungsgemäß bevorzugte kosmetische Öle sind ausgewählt aus Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls besonders bevorzugt sind flüchtige lineare Siliconöle, insbesondere Hexamethyldisiloxan (L₂), Octamethyltrisiloxan (L₃), Decamethyltetrasiloxan (L₄) sowie beliebige Zweier- und Dreiermischungen aus L₂, L₃ und/oder L₄. Flüchtige Siliconöle sind hervorragend geeignete Trägeröle für erfindungsgemäß bevorzugte Antitranspirant-Zusammensetzungen, da sie ein angenehmes Hautgefühl und eine geringe Kleideranschmutzung verleihen. Erfindungsgemäß besonders bevorzugte Antitranspirant-Zusammensetzungen sind daher durch einen Gehalt an mindestens einem flüchtigen Siliconöl von 30 - 70 Gew.-%, bevorzugt 40 - 60 Gew.-%, besonders bevorzugt 45 - 55 Gew.-%, außerordentlich bevorzugt von 50 - 53 Gew.-%, jeweils bezogen auf ihr Gesamtgewicht, gekennzeichnet.

Neben oder anstelle des mindestens einen flüchtigen Siliconöls kann auch mindestens ein flüchtiges Nichtsiliconöl enthalten sein. Bevorzugte flüchtige Nichtsiliconöle sind ausgewählt aus C₈-C₁₆-Isoparaffinen, insbesondere aus Isodecan, Isododecan, Isotetradecan und Isohexadecan sowie Mischungen hiervon. Auch dieses mindestens eine flüchtige Nichtsiliconöl ist bevorzugt in einer Gesamtmenge von 30 - 70 Gew.-%, besonders bevorzugt 40 - 60 Gew.-%, weiterhin sehr bevorzugt 45 - 55 Gew.-%, außerordentlich bevorzugt von 50 - 53 Gew.-%, jeweils bezogen auf das Gesamtgewicht der Zusammensetzung, enthalten.

Neben den vorgenannten, üblicherweise als "flüchtigen" Siliconölen bezeichneten Substanzen sowie neben den vorgenannten flüchtigen Nichtsiliconölen können erfindungsgemäß besonders bevorzugte Antitranspirant-Zusammensetzungen weiterhin mindestens ein nichtflüchtiges kosmetisches Öl, ausgewählt aus nichtflüchtigen Siliconölen und nichtflüchtigen Nichtsiliconölen, enthalten. Das nichtflüchtige Öl gleicht den negativen Effekt des flüchtigen Öls auf das Rückstandsverhalten erfindungsgemäß bevorzugter Antitranspirant-Zusammensetzungen aus. Durch die relativ schnelle Verdunstung der flüchtigen Öle können feste, unlösliche Bestandteile, insbesondere Antitranspirantwirkstoffe und Füllstoffe, auf der Haut als unschöner Rückstand sichtbar werden. Diese Rückstände können erfolgreich mit einem nichtflüchtigen Öl maskiert werden. Außerdem können mit einem Gemisch aus nichtflüchtigem und flüchtigem Öl Parameter wie Hautgefühl, Abrieb und Stabilität feingesteuert und besser an die Bedürfnisse der Verbraucher angepasst werden.

Selbstverständlich ist es ebenfalls möglich, Antitranspirant-Zusammensetzungen mit einem geringen Anteil an flüchtigen Ölen oder sogar ohne flüchtige Öle zu formulieren.

Bevorzugte nichtflüchtige Siliconöle sind ausgewählt aus höhermolekularen linearen Dimethylpolysiloxanen, im Handel erhältlich z. B. unter der Bezeichnung Dow Corning^{®} 200 Fluid mit kinematischen Viskositäten (25°C) im Bereich von 5 - 100 cSt, bevorzugt 5 - 50 cSt oder auch 5 - 10 cSt, und Baysilon^{®} 350 M (mit einer kinematischen Viskosität (25°C) von etwa 350 cSt. Erfindungsgemäß ebenfalls bevorzugte nichtflüchtige Siliconöle sind ausgewählt aus Siliconen der Formel (Sil-1), wobei x ausgewählt ist aus ganzen Zahlen von 1 - 20, bevorzugt 1 - 3.

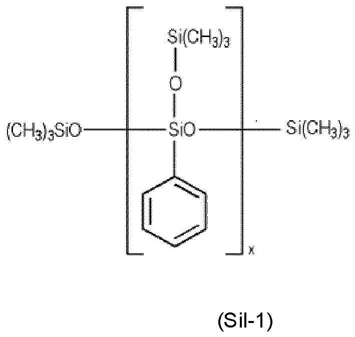

Ein bevorzugtes Siliconöl der Formel (Sil-1) ist erhältlich unter der INCl-Bezeichnung Phenyl Trimethicone.

Natürliche und synthetische Kohlenwasserstoffe, wie beispielsweise Paraffinöle, C₁₈-C₃₀-Isoparaffine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von BASF) gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD. Derartige Benzoesäureesteröle sind besonders gut zur Maskierung von Antitranspirant-Wirkstoff-Rückständen geeignet, da ihr Brechungsindex den besonders wirksamen Aluminium-Zirkonium-Mischsalzen sehr nahe kommt. Außerdem weisen diese Öle ein angenehmes Hautgefühl auf. Außerdem ermöglichen die Benzoesäureester-Öle aufgrund ihrer Polarität eine gute Wirkstofffreisetzung von wasserlöslichen Antitranspirant-Wirkstoffen aus der Fettphase der erfindungsgemäßen Zusammensetzungen heraus.

Weitere erfindungsgemäß bevorzugte nichtflüchtige Nichtsiliconöle sind ausgewählt aus verzweigten gesättigten oder ungesättigten Fettalkoholen mit 6 - 30 Kohlenstoffatomen.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (BASF) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (BASF) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Estern der linearen oder verzweigten gesättigten oder nichtflüchtige Nichtsiliconöle ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Düsotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige C₈₋₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige C₃₋₂₂-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere erfindungsgemäß besonders bevorzugte nichtflüchtige Nichtsiliconöle sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂₋C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.

Weitere erfindungsgemäß besonders bevorzugte nichtflüchtige Nichtsiliconöle sind ausgewählt aus den symmetrischen, unsymmetrischen oder cyclischen Estern der Kohlensäure mit Fettalkoholen, z. B. Glycerincarbonat, Dicaprylylcarbonat, Di-(2-ethylhexyl)carbonat oder die Ester gemäß der Lehre der DE 19756454 A1.

Weitere Öle, die erfindungsgemäß bevorzugt sein können, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.

Es kann erfindungsgemäß bevorzugt sein, Mischungen der vorgenannten Öle einzusetzen.

Bevorzugte erfindungsgemäße Antitranspirant-Zusammensetzungen sind dadurch gekennzeichnet, dass das unter Normalbedingungen flüssige Trägeröl ausgewählt ist flüchtigen Siliconölen, nichtflüchtigen Siliconölen, flüchtigen Kohlenwasserstoffölen, verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.

Es kann erfindungsgemäß außerordentlich bevorzugt sein, Mischungen der vorgenannten Öle einzusetzen, um eine optimale Feinabstimmung der Produkteigenschaften, insbesondere des Rückstandsverhaltens oder der Wirkstofffreisetzung, zu erzielen.

Weitere erfindungsgemäß bevorzugte Antitranspirant-Zusammensetzungen, insbesondere solche in Form von Stiften und Soft Solids, sind dadurch gekennzeichnet, dass sie mindestens eine unter Normalbedingungen feste Fettkomponente mit einem Schmelzpunkt von mehr als 50 - 120 °C enthalten.

Bevorzugte Fettkomponenten mit einem Schmelzpunkt von > 50 - 120 °C sind ausgewählt aus Wachsen. Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse, insbesondere Polyethylenwachse, und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren. Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäß bevorzugten Zusammensetzungen als besonders vorteilhaft erwiesen, weil sie ausgezeichnete sensorische Eigenschaften und - den Stiften - eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben.

Insbesondere können als Wachskomponente C₁₆₋₃₆-Alkylstearate und C₁₈₋₃₈-Alkylhydroxystearoylstearate, C₂₀₋₄₀-Alkylerucate sowie Cetearylbehenat bevorzugt sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Eine weitere besonders bevorzugte Ausführungsform der Erfindung enthält als Wachskomponente Cetearylbehenat, d. h. Mischungen aus Cetylbehenat und Stearylbehenat. Weitere bevorzugte Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C). Erfindungsgemäß ist als Wachskomponente hydriertes Rizinusöl, erhältlich z. B. als Handelsprodukt Cutina^{®} HR, besonders bevorzugt.

Die erfindungsgemäßen Stifte enthalten zur Verbesserung der Stiftkonsistenz und der sensorischen Eigenschaften, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs. Erfindungsgemäß bevorzugte wasserunlösliche teilchenförmige Füllstoffe sind ausgewählt aus Talkum, Siliciumdioxid, Kieselsäuren, z. B. Aerosil^{®}-Typen, Polyalkylsesquisiloxan-Partikeln (insbesondere Aerosil^{®} R972 und Aerosil^{®} 200V von Degussa), Kieselgelen, Silica, Cellulosepulvern, Stärken, modifizierten Stärken (z. B. aus Mais, Reis, Kartoffeln) und Stärkederivaten, die vorverkleistert sein können, Kaolin, Magnesiumaluminiumsilikaten, Bornitrid, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Besonders bevorzugte wasserunlösliche teilchenförmige Füllstoffe sind ausgewählt aus Talkum, Siliciumdioxid, Kieselsäuren, z. B. Aerosil^{®}-Typen, Polyalkylsesquisiloxan-Partikeln (insbesondere Aerosil^{®} R972 und Aerosil^{®} 200V von Degussa), Kieselgelen, Silica, sowie Mischungen dieser Substanzen. Außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs, ausgewählt aus Talkum, Siliciumdioxid, Kieselsäuren, Polyalkylsesquisiloxan-Partikel, Kieselgel, Silica, sowie Mischungen dieser Substanzen. Weitere außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% mindestens eines wasserunlöslichen teilchenförmigen Füllstoffs, ausgewählt aus Talkum. Weitere außerordentlich bevorzugte erfindungsgemäße Stifte enthalten, jeweils bezogen auf ihr Gesamtgewicht, insgesamt 0,5 - 6 Gew.-%, bevorzugt 1 - 5 Gew.-%, besonders bevorzugt 2 - 4 Gew.-%, außerordentlich bevorzugt 3 - 3,5 Gew.-% Talkum als einzigem wasserunlöslichen teilchenförmigen Füllstoff.

In den erfindungsgemäßen Antitranspirant-Zusammensetzungen mit einem Gehalt an freiem Wasser von 0 - 6 Gew.-%, bezogen auf das Gesamtgewicht der Zusammensetzung, ist der Antitranspirant-Wirkstoff in mindestens einem unter Normalbedingungen flüssigen Öl suspendiert. Zur besseren Anwendbarkeit wird dieser Suspension noch mindestens ein lipophiles Verdickungsmittel als Suspendierhilfe zugesetzt. Weitere bevorzugte erfindungsgemäße Antitranspirant-Zusammensetzungen, insbesondere Spray-Suds und Roll-on-Zusammensetzungen, enthalten daher mindestens ein lipophiles Verdickungsmittel. Erfindungsgemäß bevorzugte lipophile Verdickungsmittel sind ausgewählt aus hydrophobierten Tonmineralien, pyrogenen Kieselsäuren, Ethylene/Propylene/Styrene Copolymeren, Butylene/ Ethylene/Styrene Copolymeren, Dextrinestern und Siliconelastomeren. Hierunter sind hydrophobierte Tonmineralien besonders bevorzugt. Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Die Gelbildung erfolgt durch den Zusatz geringer Mengen an Aktivatoren, wie insbesondere Ethanol oder Propylencarbonat, aber auch Wasser. Derartige Gele sind beispielsweise unter der Handelsbezeichnung Bentone^{®} oder Thixogel erhältlich. Bevorzugte erfindungsgemäße Zusammensetzungen enthalten mindestens einen Aktivator in einer Gesamtmenge von 0,1 - 3 Gew.-%, bevorzugt 0,3 - 1,6 Gew.-%, jeweils bezogen auf das Gesamtgewicht der treibmittelfreien erfindungsgemäßen Zusammensetzung. Weitere bevorzugte erfindungsgemäße Zusammensetzungen enthalten mindestens einen Aktivator, ausgewählt aus Ethanol, Propylencarbonat und Wasser sowie Mischungen hiervon, in einer Gesamtmenge von 0,1 - 3 Gew.-%, bevorzugt 0,3 - 1,6 Gew.-%, jeweils bezogen auf das Gesamtgewicht der treibmittelfreien erfindungsgemäßen Zusammensetzung. Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der treibmittelfreien erfindungsgemäßen Zusammensetzung, enthalten.

Weitere erfindungsgemäß bevorzugte lipophile Verdickungsmittel sind ausgewählt aus pyrogenen Kieselsäuren, z. B. den Handelsprodukten der Aerosil^{®}-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Antitranspirant-Zusammensetzungen mit einem Gehalt an freiem Wasser von 0 - 6 Gew.-%, bezogen auf das Gesamtgewicht der Zusammensetzung, enthalten mindestens eine pyrogene Kieselsäure, bevorzugt mindestens eine hydrophobierte pyrogene Kieselsäure, in einer Gesamtmenge von 0,5 - 10 Gew.-%, bevorzugt 0,8 - 5 Gew.-%, besonders bevorzugt 1 - 4 Gew.-%, außerordentlich bevorzugt 1,5 - 2 Gew.-%, jeweils bezogen auf das Gesamtgewicht der treibmittelfreien erfindungsgemäßen Zusammensetzung. Weitere erfindungsgemäß bevorzugte Antitranspirant-Zusammensetzungen mit einem Gehalt an freiem Wasser von 0 - 6 Gew.-%, bezogen auf das Gesamtgewicht der Zusammensetzung, insbesondere Spray-Suds und Roll-on-Zusammensetzungen, enthalten mindestens eine hydrophobierte pyrogene Kieselsäure und mindestens eine hydrophile Kieselsäure.

Bevorzugte erfindungsgemäße Antitranspirant-Zusammensetzungen mit einem Gehalt an freiem Wasser von 0 - 6 Gew.-%, bezogen auf das Gesamtgewicht der Zusammensetzung, enthalten mindestens einen Polyethylenglycolether eines linearen oder verzweigten C₁₀ - C₂₂-Alkanols mit 8 - 150 Ethylenoxideinheiten enthalten ist. Diese Verbindungen sind als Öl-in-Wasser-Emulgatoren bekannt.

Überraschend wurde festgestellt, dass der Zusatz derartiger Polyethylenglycolether eines linearen oder verzweigten C₁₀ - C₂₂-Alkanols mit 8 - 150 Ethylenoxideinheiten zu einer stark verbesserten Wirkstofffreisetzung des schweißhemmenden Wirkstoffs führt. Besonders überraschend war, dass längst nicht alle Öl-in-Wasser-Emulgatoren diesen begünstigenden Effekt auf die erfindungsgemäßen Zusammensetzungen ausüben, auch nicht solche, die im selben HLB-Wertbereich liegen wie die erfindungsgemäß bevorzugten Polyethylenglycolether eines linearen oder verzweigten C₁₀ - C₂₂-Alkanols mit 8 - 150 Ethylenoxideinheiten.

Bevorzugte erfindungsgemäße Zusammensetzungen sind dadurch gekennzeichnet, dass der mindestens eine Polyethylenglycolether eines linearen oder verzweigten C₁₀ - C₂₂-Alkanols mit 8 bis 150 Ethylenoxideinheiten ausgewählt ist aus Laureth-20, Laureth-25, Laureth-30, Laureth-35, Laureth-40, Laureth-45, Laureth-50, Laureth-55, Laureth-60, Laureth-65, Laureth-70, Laureth-75, Laureth-80, Laureth-85, Laureth-90, Laureth-95, Laureth-100, Myristeth-20, Myristeth-25, Myristeth-30, Myristeth-35, Myristeth-40, Myristeth-45, Myristeth-50, Myristeth-55, Myristeth-60, Myristeth-65, Myristeth-70, Myristeth-75, Myristeth-80, Myristeth-85, Myristeth-90, Myristeth-95, Myristeth-100, Ceteth-20, Ceteth-25, Ceteth-30, Ceteth-35, Ceteth-40, Ceteth-45, Ceteth-50, Ceteth-55, Ceteth-60, Ceteth-65, Ceteth-70, Ceteth-75, Ceteth-80, Ceteth-85, Ceteth-90, Ceteth-95, Ceteth-100, Ceteth-110, Ceteth-120, Ceteth-130, Ceteth-140, Ceteth-150, Isoceteth-20, Isoceteth-25, Isoceteth-30, Isoceteth-35, Isoceteth-40, Isoceteth-45, Isoceteth-50, Isoceteth-55, Isoceteth-60, Isoceteth-65, Isoceteth-70, Isoceteth-75, Isoceteth-80, Isoceteth-85, Isoceteth-90, Isoceteth-95, Isoceteth-100, Isoceteth-110, Isoceteth-120, Isoceteth-130, Isoceteth-140, Isoceteth-150, Steareth-20, Steareth-25, Steareth-30, Steareth-35, Steareth-40, Steareth-45, Steareth-50, Steareth-55, Steareth-60, Steareth-65, Steareth-70, Steareth-75, Steareth-80, Steareth-85, Steareth-90, Steareth-95, Steareth-100, Steareth-110, Steareth-120, Steareth-130, Steareth-140, Steareth-150, Isosteareth-20, Isosteareth-25, Isosteareth-30, Isosteareth-35, Isosteareth-40, Isosteareth-45, Isosteareth-50, Isosteareth-55, Isosteareth-60, Isosteareth-65, Isosteareth-70, Isosteareth-75, Isosteareth-80, Isosteareth-85, Isosteareth-90, Isosteareth-95, Isosteareth-100, Isosteareth-110, Isosteareth-120, Isosteareth-130, Isosteareth-140, Isosteareth-150, Arachideth-20, Arachideth-25, Arachideth-30, Arachideth-35, Arachideth-40, Arachideth-45, Arachideth-50, Arachideth-55, Arachideth-60, Arachideth-65, Arachideth-70, Arachideth-75, Arachideth-80, Arachideth-85, Arachideth-90, Arachideth-95, Arachideth-1 00, Arachideth-110, Arachideth-120, Arachideth-130, Arachideth-140, Arachideth-150, Beheneth-20, Beheneth-25, Beheneth-30, Beheneth-35, Beheneth-40, Beheneth-45, Beheneth-50, Beheneth-55, Beheneth-60, Beheneth-65, Beheneth-70, Beheneth-75, Beheneth-80, Beheneth-85, Beheneth-90, Beheneth-95, Beheneth-100, Beheneth-110, Beheneth-120, Beheneth-130, Beheneth-140, Beheneth-150, sowie Mischungen hiervon. Außerordentlich bevorzugt sind Ceteth-12, Steareth-12, Ceteareth-12 sowie Steareth-1 00.

Bevorzugte erfindungsgemäße Antitranspirant-Zusammensetzungen mit einem Gehalt an freiem Wasser von 0 - 6 Gew.-%, bezogen auf das Gesamtgewicht der Zusammensetzung, enthalten mindestens einen Polyethylenglycolether eines linearen oder verzweigten C₁₀ C₂₂-Alkanols mit 8 - 150 Ethylenoxideinheiten in einer Gesamtmenge von 0,1 - 4 Gew.-%, bevorzugt 0,5 - 3,5, besonders bevorzugt 1 - 3 Gew.-%, außerordentlich bevorzugt 1,5 - 2,6 Gew.-%, jeweils bezogen auf das Gesamtgewicht der Zusammensetzung.

Bevorzugte erfindungsgemäße Antitranspirant-Zusammensetzungen mit einem Gehalt an freiem Wasser von 0 - 6 Gew.-%, bezogen auf das Gesamtgewicht der Zusammensetzung und einer Viskosität von 100 bis 15.000 mPas bei 20 °C werden mittels eines komprimierten Treibmittels als Spray aus einem Druckbehälter ausgetragen.

Erfindungsgemäß bevorzugte Treibmittel (Treibgase) sind ausgewählt aus Propan, Propen, n-Butan, iso-Butan, iso-Buten, n-Pentan, Penten, iso-Pentan, iso-Penten, Methan, Ethan, Dimethylether, Stickstoff, Luft, Sauerstoff, Lachgas, Dichlorfluormethan, Chlordifluormethan, Chlorfluormethan, 1,1,2,2-Tetrachlor-1-fluorethan, 1,1,1,2-Tetrachlor-2-fluorethan, 1,2,2-Trichlor-1,1-difluorethan, 1,1,2-Trichlor-1,2-difluorethan, 1,1,1-Trichlor-2,2-difluorethan, 2,2-Dichlor-1,1,1-trifluorethan, 1,2-Dichlor-1,1,2-trifluorethan, 2-Chlor-1,1,1,2-tetrafluorethan, 1-Chlor-1,1,2,2-tetrafluorethan, 1,1,2-Trichlor-2-fluorethan, 1,2-Dichlor-1,2-difluorethan, 1,2-Dichlor-1,1-difluorethan, 1-Chlor-1,2,2-trifluorethan, 2-Chlor-1,1,1-trifluorethan, 1-Chlor-1,1,2-trifluorethan, 1,2-Dichlor-1-fluorethan, 1,1-Dichlor-1-fluorethan, 2-Chlor-1,1-difluorethan, 1-Chlor-1,1-difluorethan, 1-Chlor-2-fluorethan, 1-Chlor-1-fluorethan, 2-Chlor-1,1-difluorethen, 1,1,1,3-Tetrafluorethan, Heptafluoro-n-propan, Perfluorethan, Monochlordifluormethan, 1,1-Difluorethan, und zwar sowohl einzeln als auch in Kombination.

Besonders bevorzugt sind Propan, n-Butan, iso-Butan sowie Mischungen dieser Treibmittel. Es hat sich auch gezeigt, dass der Einsatz von n-Butan als einzigem Treibmittel erfindungsgemäß besonders bevorzugt sein kann. Weiterhin bevorzugt sind auch 1,1-Difluorethan, Propan, n-Butan, iso-Butan sowie Mischungen dieser Treibmittel, insbesondere Mischungen aus 1,1-Difluorethan und n-Butan.

Das Gesamtgewicht an mindestens einem organischen Treibmittel beträgt bevorzugt 10 - 90 Gew.-%, besonders bevorzugt 40 - 85 Gew.-%, außerordentlich bevorzugt 50 - 80 Gew.-% und weiter bevorzugt 70, 72, 74, 76, 78, 82, 84 oder 86 Gew.-%, jeweils bezogen auf das Gesamtgewicht der Zubereitung, bestehend aus der erfindungsgemäßen Antitranspirant-Zusammensetzung und dem Treibmittel.

Auch Kohlendioxid, Luft, Sauerstoff, Stickstoff, Helium und Argon können im Sinne der vorliegenden Erfindung vorteilhaft als Treibmittel eingesetzt werden. Ihr Anteil wird im Vergleich zu den vorstehend genannten organischen Treibmitteln deutlich geringer gewählt und beträgt 1 - 10 Gew.-%, bevorzugt 2 - 9 Gew.-%, jeweils bezogen auf das Gesamtgewicht der Zubereitung, bestehend aus der erfindungsgemäßen Antitranspirant-Zusammensetzung und dem Treibmittel. Als Behälter, insbesondere als Druckgasbehälter (für den Einsatz mit komprimiertem Treibmittel) sind Gefäße aus Metall (Aluminium, Weißblech, Zinn), geschütztem bzw. nicht-splitterndem Kunststoff oder aus Glas, das außen mit Kunststoff beschichtet ist, bevorzugt, bei deren Auswahl Druck- und Bruchfestigkeit, Korrosionsbeständigkeit, Befüllbarkeit wie auch ästhetische Gesichtspunkte, Handlichkeit, Bedruckbarkeit etc. eine Rolle spielen. Bevorzugt gewährleisten spezielle Innenschutzlacke die Korrosionsbeständigkeit.

Bevorzugte erfindungsgemäße Antitranspirant-Zusammensetzungen, die, jeweils bezogen auf ihr Gesamtgewicht, einen Gehalt an freiem Wasser von mehr als 6 bis 96 Gew.-%, bevorzugt 15 bis 85 Gew.-%, besonders bevorzugt 30 bis 75 Gew.-%, außerordentlich bevorzugt 50 bis 70 Gew.-%, aufweisen, liegen als Öl-in-Wasser-Emulsion, die keine Mikroemulsion darstellt, vor und enthalten 0,5 - 6,5 Gew.-% Öl- oder Fettphase, umfassend mindestens eine bei 20 °C flüssige Ölkomponente, ausgewählt aus linearen und verzweigten gesättigten ein- oder mehrwertigen C₃ - C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, mindestens einen nichtionischen Emulgator mit einem HLB-Wert im Bereich von 3 - 6 in einer Gesamtmenge von 1,8 - 3 Gew.-%, mindestens einen nichtionischen Emulgator mit einem HLB-Wert im Bereich von 12 - 18 in einer Gesamtmenge von 1 - 2 Gew.-%, und mindestens ein Polysaccharid in einer Gesamtmenge von 0,01 - 1,0 Gew.-%, bevorzugt 0,05 - 0,5 und besonders bevorzugt 0,09 - 0,2 Gew.-%, jeweils bezogen auf das Gesamtgewicht der Zusammensetzung. Besonders bevorzugt enthalten diese Öl-in-Wasser-Emulsionen 60 bis 96 Gew.-%, bevorzugt 65 - 75 Gew.-%, freies Wasser, jeweils bezogen auf ihr Gesamtgewicht. Unter erfindungsgemäß geeigneten Polysacchariden werden sowohl nicht-modifizierte Polysaccharide, wie beispielsweise Xanthan oder Stärke, als auch chemisch modifizierte Polysaccharid-Derivate, wie beispielsweise Aluminiumstärkeoctenylsuccinat, Hydroxypropylmethylcellulose oder dehydratisiertes Xanthan (INCI: Dehydroxanthan Gum), als auch physikalisch modifizierte Polysaccharide, beispielsweise eine durch thermische Behandlung vorverkleisterte Stärke, verstanden.

Erfindungsgemäß bevorzugte Polysaccharide sind ausgewählt aus Stärken, insbesondere aus Mais, Kartoffeln und Weizen, deren Bestandteilen wie Amylose und Amylopektin, Stärkehydrolysaten und Stärkeabbauprodukten, wie Maltodextrin, den physikalisch oder chemisch modifizierten Stärkederivaten, insbesondere den anionischen Stärkederivaten Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken und Natriumstärkeglycolat, Cellulose, den chemisch modifizierten Cellulosederivaten Methylcellulose, Hydroxypropylcellulose, Hydroxyethylcellulose, Hydroxypropylmethylcellulose, Hydroxypropylethylcellulose, Hydroxyethylmethylcellulose und Carboxymethylcellulose. Polysaccharide, die Gums oder Gummen bilden, wie beispielsweise Guar-Gum, Xanthan-Gum, Dehydroxanthan Gum, Alginate, insbesondere Natriumalginat, Gummi arabicum, Karaya-Gummi, Carrageenane, Johannisbrotkernmehl, Leinsamen-Gums und Agar-Agar, können ebenfalls enthalten sein, sind aber weniger bevorzugt. In einer besonders bevorzugten Ausführungsform sind die erfindungsgemäßen Zusammensetzungen frei von Polysaccharid-Gums. In einer weiteren besonders bevorzugten Ausführungsform sind die erfindungsgemäßen Zusammensetzungen frei von Guar-Gum, Xanthan-Gum, Dehydroxanthan Gum, Alginate, insbesondere Natriumalginat, Gummi arabicum, Karaya-Gummi, Carrageenane, Johannisbrotkernmehl, Leinsamen-Gums und Agar-Agar. Besonders bevorzugte erfindungsgemäße Öl-in-Wasser-Emulsionen enthalten mindestens ein Polysaccharid, ausgewählt aus anionischen und nichtionischen Polysacchariden sowie Mischungen hiervon. Weitere besonders bevorzugte erfindungsgemäße Öl-in-Wasser-Emulsionen enthalten mindestens ein Polysaccharid, ausgewählt aus anionischen und nichtionischen Polysacchariden, die keine Polysaccharid-Gums bilden. Weitere besonders bevorzugte erfindungsgemäße Öl-in-Wasser-Emulsionen enthalten ein anionisches Polysaccharid, das ist ausgewählt aus Aluminiumstärkeoctenylsuccinat, Natriumstärkeoctenylsuccinat, Calciumstärkeoctenylsuccinat, Distärkephosphaten, Hydroxyethylstärkephosphaten, Hydroxypropylstärkephosphaten, Natriumcarboxymethylstärken, Natriumstärkeglycolat sowie Mischungen hiervon. Ein erfindungsgemäß außerordentlich bevorzugtes anionisches Polysaccharid ist Aluminiumstärkeoctenylsuccinat. Bevorzugte nichtionische Emulgatoren mit einem HLB-Wert im Bereich von 3 - 6 sind ausgewählt aus linearen gesättigten und ungesättigten C₁₂ - C₃₀-Alkanolen, die mit 1 - 4 Ethylenoxid-Einheiten pro Molekül verethert sind, besonders bevorzugt ausgewählt aus Steareth, Ceteth, Myristeth, Laureth, Trideceth, Arachideth und Beheneth mit jeweils 1 - 4 Ethylenoxid-Einheiten pro Molekül. Bevorzugte nichtionische Emulgatoren mit einem HLB-Wert im Bereich von 12 - 18 sind ausgewählt aus linearen gesättigten und ungesättigten C₁₂ - C₂₄-Alkanolen, die mit 7 - 40 Ethylenoxid-Einheiten pro Molekül verethert sind, besonders bevorzugt ausgewählt aus Steareth, Ceteth, Myristeth, Laureth, Trideceth, Arachideth und Beheneth mit jeweils 7 - 40 Ethylenoxid-Einheiten pro Molekül.

Besonders bevorzugte Antitranspirant-Zusammensetzungen liegen als Öl-in-Wasser-Emulsion, die keine Mikroemulsion darstellt, vor und enthalten, jeweils bezogen auf ihr Gesamtgewicht, 60 - 96 Gew.-%, bevorzugt 65 - 75 Gew.-%, freies Wasser, insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin 0,01 - 0,3 Gew.-%, bevorzugt 0,05 - 0,2 Gew.-%, besonders bevorzugt 0,1 - 0,15 Gew.-%, Squalan, weiterhin 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat, weiterhin insgesamt 0,1 - 5 Gew.-%, bevorzugt 0,2 - 4,5 Gew.-%, besonders bevorzugt 0,3 - 4 Gew.-%, außerordentlich bevorzugt 0,5 - 3,5 Gew.-%, einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat, weiterhin Glycerin in einer Gesamtmenge von 0,01 - 0,5 Gew.-%, bevorzugt 0,05 - 0,3 Gew.-%, besonders bevorzugt 0,1 - 0,2 Gew.-%, weiterhin Ca²⁺ in einer Gesamtmenge von 0,001 - 0,1 Gew.-%, bevorzugt 0,01 - 0,08 Gew.-%, besonders bevorzugt 0,03 - 0,06 Gew.-%, außerordentlich bevorzugt 0,04 - 0,05 Gew.-%, weiterhin 0,5 - 6,5 Gew.-% Öl- oder Fettphase, umfassend mindestens eine bei 20 °C flüssige Ölkomponente, ausgewählt aus linearen und verzweigten gesättigten ein- oder mehrwertigen C₃ - C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, mindestens einen nichtionischen Emulgator mit einem HLB-Wert im Bereich von 3 - 6 in einer Gesamtmenge von 1,8 - 3 Gew.-%, mindestens einen nichtionischen Emulgator mit einem HLB-Wert im Bereich von 12 - 18 in einer Gesamtmenge von 1 - 2 Gew.-%, und mindestens ein Polysaccharid in einer Gesamtmenge von 0,01 - 1,0 Gew.-%, bevorzugt 0,05 - 0,5 und besonders bevorzugt 0,09 - 0,2 Gew.-%.

Bevorzugte erfindungsgemäße Antitranspirant-Zusammensetzungen enthalten mindestens einen Riechstoff, besonders bevorzugt in einer Gesamtmenge von 0,1 bis 9 Gew.-%, außerordentlich bevorzugt 0,3 - 7 Gew.-%, außerordentlich bevorzugt 1 - 4 Gew.-%, jeweils bezogen auf ihr Gesamtgewicht.

Um die schweißhemmende und geruchsreduzierende Wirkung der erfindungsgemäßen Antitranspirant-Zusammensetzungen weiter zu verbessern, enthalten diese in einer bevorzugten Ausführungsform mindestens einen Deodorant-Wirkstoff.

Erfindungsgemäß bevorzugte Deodorant-Wirkstoffe sind keimhemmende Mittel, präbiotisch wirksame Komponenten sowie Enzyminhibitoren oder, besonders bevorzugt, Kombinationen der genannten Wirkstoffe.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzethoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in DE 10333245 und DE 102004011968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.

Weitere bevorzugte Deodorant-Wirkstoffe sind ausgewählt aus den keimhemmend wirkenden Parfümölen und den Deosafe^{®}-Parfümölen, die von der Firma Symrise, vormals Haarmann und Reimer, erhältlich sind.

Weitere bevorzugte Deodorant-Wirkstoffe sind ausgewählt aus Silbersalzen, insbesondere Silbercitrat, Dihydrogensilbercitrat, Silberlactat und Silbersulfat, löslichen Komplexsalzen des Silbers, kolloidalem Silber und Silberzeolithen.

Zu den Enzyminhibitoren gehören Stoffe, die die für die Schweißzersetzung verantwortlichen Enzyme, insbesondere die Arylsulfatase, β-Glucuronidase, Aminoacylase, Esterasen, Lipasen und/oder Lipoxigenase, hemmen, z. B. Trialkylcitronensäureester, insbesondere Triethylcitrat, oder Zinkglycinat.

Bevorzugte erfindungsgemäße Antitranspirant-Zusammensetzungen sind dadurch gekennzeichnet, dass der mindestens eine Deodorant-Wirkstoff ausgewählt ist aus Arylsulfatase-Inhibitoren, beta-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere alpha-(2-Ethylhexyl)glycerinether, Phenoxyethanol, keimhemmend wirkenden Parfümölen, Cyclodextrinen, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Silbersalzen, Silberzeolithen sowie Mischungen der vorgenannten Substanzen.

Weitere bevorzugte erfindungsgemäße Antitranspirant-Zusammensetzungen enthalten, jeweils bezogen auf ihr Gesamtgewicht, mindestens einen Deodorant-Wirkstoff in einer Gesamtmenge von 0,1 - 10 Gew.-%, bevorzugt 0,2 - 7 Gew.-%, besonders bevorzugt 0,3 - 5 Gew.-% und außerordentlich bevorzugt 0,4 - 1,0 Gew.-%.

Eine weitere bevorzugte Ausführungsform der Erfindung ist dadurch gekennzeichnet, dass 0,01 - 5 Gew.-%, bevorzugt 0,05 - 2 Gew.-%, besonders bevorzugt 0,1 - 1 Gew.-%, jeweils bezogen auf die gesamte erfindungsgemäße Zusammensetzung, einer aus einem natürlichen Mineralwasser, einem Thermalwasser oder einem natürlichen Heilwasser erhaltenen Mineralstoffmischung enthalten ist. Überraschend wurde festgestellt, dass derartige Mineralstoffmischungen die schweißhemmende Leistung der erfindungsgemäßen Zusammensetzungen weiter verbessern können. Außerdem können sich derartige Mineralstoffmischungen günstig auf die Hautverträglichkeit der erfindungsgemäßen Zusammensetzungen auswirken. Die Bezeichnung "natürliches Mineralwasser" richtet sich nach der Definition der deutschen Mineral- und Tafelwasserverordnung (MinTafWV, § 2). Als Mineralstoffmischung gilt der feste Rückstand der genannten Wässer. Erfindungsgemäß besonders bevorzugte Mineralstoffmischungen stammen aus dem Thermalwasser von La Toja (Spanien), Karlsbad (Tschechien), Bad Blumau, Bad Radkersburg, Aachen, Wiesbaden (alle Deutschland), La Bourboule, Enghien-les-bains, Allevard-les-bains, Digne, Lons le Saunier, Nyrac-les-bains, Eaux Bonnes, Rochefort, les Fumades, Saint Christau, Uriage-les-bains, la Roche-Posay (alle Frankreich) oder den natürlichen Mineralwässern oder Heilwässern von Evian, Volvic, Vichy, Avène, Vittel (alle Frankreich), Gerolstein oder Fachingen (Deutschland).

Ein weiterer Gegenstand der vorliegenden Anmeldung ist ein Verfahren zur nicht-therapeutischen Schweißreduktion, dass dadurch gekennzeichnet ist, dass eine erfindungsgemäße oder eine erfindungsgemäß bevorzugte Antitranspirant-Zusammensetzung auf die Haut, insbesondere die Achselhaut, aufgetragen wird. Bezüglich weiterer bevorzugter Ausführungsformen des erfindungsgemäßen Verfahrens gilt mutatis mutandis das zu den erfindungsgemäßen Antitranspirant-Zusammensetzungen Gesagte.

Ein weiterer Gegenstand der vorliegenden Anmeldung ist die Verwendung einer erfindungsgemäßen oder einer erfindungsgemäß bevorzugten Antitranspirant-Zusammensetzung zur nicht-therapeutischen Schweißreduktion. Bezüglich weiterer bevorzugter Ausführungsformen der erfindungsgemäßen Verwendung gilt mutatis mutandis das zu den erfindungsgemäßen Antitranspirant-Zusammensetzungen Gesagte.

### Experimenteller Teil

Für jeden Test wurde auf jeweils drei Ganzhautmodelle (hergestellt gemäß Mewes et al. (2007) Skin Pharmacol Physiol 20:85-95) eine Placebo-Formulierung bzw. die wirkstoffhaltige Verum-Formulierung topisch appliziert und für 24 Stunden inkubiert. In den Modellen wurde anschliessend der ATP-Gehalt mit Hilfe des ATPlite 1 step-Kits (Perkin Elmer) bestimmt.

AT-Spray-Sud, Verum (Mengenangaben in Gew.-%):

| | |
|---|---|
| Cyclomethicone | 55,37 |
| Caprylic/Capric/Myristic/Stearic Triglyceride | 0,33 |
| Lauric/Myristic Triglyceride/Diglyceride (Cocoglycerides) | 0,1 |
| Stearic Acid, PalmiticAcid (1:1) | 0,1 |
| Palmitic/Stearic Triglyceride (ex Hydrogenated Vegetable Oil) | 0,1 |
| Tocopherylacetat | 0,33 |
| Squalan | 0,1 |
| Isopropyl Myristate | 6,7 |
| Disteardimonium Hectorite | 2,5 |
| Kristallwasser | 6,7 |
| Aluminum Chlorohydrate (USP) | 26,7 |
| Propylencarbonat | 0,9 |

AT-Spray-Sud, Placebo (Mengenangaben in Gew.-%):

| | |
|---|---|
| Cyclomethicone | 56,5 |
| Caprylic/Capric/Myristic/Stearic Triglyceride | - |
| Lauric/Myristic Triglyceride/Diglyceride (Cocoglycerides) | - |
| Stearic Acid, Palmitic Acid (1:1) | - |
| Palmitic/Stearic Triglyceride (ex Hydrogenated Vegetable Oil) | - |
| Tocopherylacetat | - |
| Squalan | - |
| Isopropyl Myristate | 6,7 |
| Disteardimonium Hectorite | 2,5 |
| Kristallwasser | 6,7 |
| Aluminum Chlorohydrate (USP) | 26,7 |
| Propylencarbonat | 0,9 |

AT-Roll-on, Placebo (Mengenangaben in Gew.-%):

| | |
|---|---|
| Aluminum Chlorohydrate (USP) | 16 |
| Kristallwasser | 4 |
| Steareth-2 | 2,5 |
| Steareth-21 | 1,2 |
| PPG-15 Stearyl Ether | 1,0 |
| Aluminum Starch Octenylsuccinate | 0,1 |
| Tetrasodium EDTA | 0,1 |
| Pentaerythrityl Tetra-di-t-butyl Hydroxyhydrocinnamate | 0,01 |
| Wasser | 75,09 |

AT-Roll-on, Verum (Mengenangaben in Gew.-%):

| | |
|---|---|
| Aluminum Chlorohydrate (USP) | 16 |
| Kristallwasser | 4 |
| Steareth-2 | 2,5 |
| Steareth-21 | 1,2 |
| PPG-15 Stearyl Ether | 1,0 |
| Caprylic/Capric/Myristic/Stearic Triglyceride | 0,2 |
| Tocopherylacetat | 0,2 |
| Glycerin | 0,1 |
| Lauric/Myristic Triglyceride/Diglyceride (Cocoglycerides) | 0,1 |
| Squalan | 0,1 |
| Aluminum Starch Octenylsuccinate | 0,1 |
| Tetrasodium EDTA | 0,1 |
| Pentaerythrityl Tetra-di-t-butyl Hydroxyhydrocinnamate | 0,01 |
| Calciumchlorid | 0,01 |
| Wasser | 74,38 |

Testlösung C (Mengenangaben in Gew.-%):

| | |
|---|---|
| Caprylic/Capric/Myristic/Stearic Triglyceride | 0,2 |
| Tocopheryl Acetate | 0,2 |
| Lauric/Myristic Triglyceride/Diglyceride (Cocoglycerides) | 0,1 |
| Squalane | 0,1 |
| Glycerin | 0,1 |
| Calcium Chloride | 0,01 |
| Dimethylsulfoxid | 52,0 |
| Wasser | 47,2 |

Ergebnisse der ATP-Messungen

| Probenbezeichnung | ATP-Gehalt [%] | Standardabweichung (%-Punkte) |
|---|---|---|
| AT-Spray-Sud, Placebo | 100 | 16 |
| AT-Spray-Sud, Verum | 3132 | 277 |
| AT-Roll-on, Placebo | 100 | 41 |
| AT-Roll-on, Verum | 223 | 95 |
| 1 Volumenteil Wasser + 1 Volumenteil Dimethylsulfoxid (Placebo) | 100 | 22 |
| Testlösung C (Verum) | 156 | 28 |

Die erfindungsgemäßen Zusammensetzungen zeigten einen deutlich erhöhten ATP-Gehalt in den Zellen.

## Patentansprüche

1. Kosmetische Antitranspirant-Zusammensetzung, enthaltend:
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,01 - 0,3 Gew.-% Squalan, weiterhin
c) insgesamt 0,01 - 0,5 Gew.-%, mindestens einer Substanz, ausgewählt aus Tocopherolen und Tocopherylestern sowie Mischungen hiervon, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%,
wobei sich, sofern nichts anderes vermerkt ist, alle Gew.-%-Angaben auf das Gesamtgewicht der Antitranspirant-Zusammensetzung ohne Berücksichtigung ggf. vorhandener Treibmittel beziehen.

2. Kosmetische Antitranspirant-Zusammensetzung gemäß Anspruch 1, **dadurch gekennzeichnet, dass** die Zusammensetzung 0 bis 6 Gew.-% freies Wasser enthält, bezogen auf das Gewicht der gesamten Zusammensetzung.

3. Kosmetische Antitranspirant-Zusammensetzung gemäß Anspruch 1, **dadurch gekennzeichnet, dass** die Zusammensetzung mehr als 6 Gew.-% bis 96 Gew.-% freies Wasser enthält, bezogen auf das Gewicht der gesamten Zusammensetzung.

4. Kosmetische Antitranspirant-Zusammensetzung gemäß Anspruch 1 oder 2, **dadurch gekennzeichnet, dass**, bezogen auf das Gewicht der gesamten Zusammensetzung, insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen enthalten ist.

5. Kosmetische Antitranspirant-Zusammensetzung gemäß Anspruch 3, **dadurch gekennzeichnet, dass**, bezogen auf das Gewicht der gesamten Zusammensetzung, 0,01 bis 0,5 Gew.-% Glycerin enthalten ist.

6. Kosmetische Antitranspirant-Zusammensetzung gemäß Anspruch 3 oder 5, **dadurch gekennzeichnet, dass**, bezogen auf das Gewicht der gesamten Zusammensetzung, 0,001 bis 0,1 Gew.-% Ca²⁺ enthalten ist.

7. Kosmetische Antitranspirant-Zusammensetzung gemäß Anspruch 1, 2 oder 4, **dadurch gekennzeichnet, dass** die gesättigte, unsubstituierte Alkansäure mit 12 bis 18 Kohlenstoffatomen ausgewählt ist aus n-Dodecansäure, n-Tridecansäure, n-Tetradecansäure, n-Pentadecansäure, n-Hexadecansäure (Palmitinsäure), n-Heptadecansäure und n-Octadecansäure (Stearinsäure) sowie Mischungen hiervon, bevorzugt ausgewählt ist aus n-Dodecansäure, n-Tetradecansäure, n-Hexadecansäure (Palmitinsäure) und n-Octadecansäure (Stearinsäure) sowie Mischungen hiervon, besonders bevorzugt ausgewählt ist aus n-Hexadecansäure (Palmitinsäure) und n-Octadecansäure (Stearinsäure) sowie Mischungen hiervon, außerordentlich bevorzugt aus einer Mischung von n-Hexadecansäure (Palmitinsäure) und n-Octadecansäure (Stearinsäure) im Molverhältnis 1:1.

8. Kosmetische Antitranspirant-Zusammensetzung gemäß Anspruch 1, 2, 4 oder 7, **dadurch gekennzeichnet, dass** sie, jeweils bezogen auf ihr Gesamtgewicht, enthält:
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,01 - 0,3 Gew.-%, bevorzugt 0,05 - 0,2 Gew.-%, besonders bevorzugt 0,1 - 0,15 Gew.-%, Squalan, weiterhin
c) insgesamt 0,01 - 0,5 Gew.-%, mindestens einer Substanz, ausgewählt aus Tocopherolen und Tocopherylestern sowie Mischungen hiervon, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%,
e) 0 bis 6 Gew.-% freies Wasser,
f) insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, die bevorzugt ausgewählt ist aus n-Dodecansäure, n-Tridecansäure, n-Tetradecansäure, n-Pentadecansäure, n-Hexadecansäure (Palmitinsäure), n-Heptadecansäure und n-Octadecansäure (Stearinsäure) sowie Mischungen hiervon, bevorzugt ausgewählt ist aus n-Dodecansäure, n-Tetradecansäure, n-Hexadecansäure (Palmitinsäure) und n-Octadecansäure (Stearinsäure) sowie Mischungen hiervon, besonders bevorzugt ausgewählt ist aus n-Hexadecansäure (Palmitinsäure) und n-Octadecansäure (Stearinsäure) sowie Mischungen hiervon, außerordentlich bevorzugt aus einer Mischung von n-Hexadecansäure (Palmitinsäure) und n-Octadecansäure (Stearinsäure) im Molverhältnis 1:1.

9. Kosmetische Antitranspirant-Zusammensetzung gemäß Anspruch 1, 2, 4, 7 oder 8, **dadurch gekennzeichnet, dass** sie als Stift, Spray-Sud oder Roll-on-Zusammensetzung konfektioniert sind und dass sie 30 - 70 Gew.-%, bezogen auf die gesamte Zusammensetzung, an mindestens einem unter Normalbedingungen flüssigen kosmetischen Öl enthalten.

10. Kosmetische Antitranspirant-Zusammensetzung gemäß Anspruch 1, 3, 5 oder 6, **dadurch gekennzeichnet, dass** sie als Öl-in-Wasser-Emulsion, die keine Mikroemulsion darstellt, vorliegt und, jeweils bezogen auf ihr Gesamtgewicht, folgendes enthält: 60 bis 96 Gew.-%, bevorzugt 65 - 75 Gew.-%, freies Wasser, insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin 0,01 - 0,3 Gew.-%, bevorzugt 0,05 - 0,2 Gew.-%, besonders bevorzugt 0,1 - 0,15 Gew.-%, Squalan, weiterhin 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat, weiterhin insgesamt 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat, weiterhin Glycerin in einer Gesamtmenge von 0,01 bis 0,5 Gew.-%, bevorzugt 0,05 bis 0,3 Gew.-%, besonders bevorzugt 0,1 bis 0,2 Gew.-%, weiterhin Ca²⁺ in einer Gesamtmenge von 0,001 bis 0,1 Gew.-%, bevorzugt 0,01 bis 0,08 Gew.-%, besonders bevorzugt 0,03 bis 0,06 Gew.-%, außerordentlich bevorzugt 0,04 bis 0,05 Gew.-%, weiterhin 0,5 - 6,5 Gew.-% Öl- oder Fettphase, umfassend mindestens eine bei 20 °C flüssige Ölkomponente, ausgewählt aus linearen und verzweigten gesättigten ein- oder mehrwertigen C₃- C₃₀-Alkanolen, die mit mindestens einer Propylenoxid-Einheit pro Molekül verethert sind, Propylenglycolmonoestern von verzweigten gesättigten C₆ - C₃₀-Alkancarbonsäuren und verzweigten gesättigten C₁₀ - C₃₀-Alkanolen, weiterhin mindestens einen nichtionischen Emulgator mit einem HLB-Wert im Bereich von 3 - 6 in einer Gesamtmenge von 1,8 - 3 Gew.-%, weiterhin mindestens einen nichtionischen Emulgator mit einem HLB-Wert im Bereich von 12 - 18 in einer Gesamtmenge von 1 - 2 Gew.-%, und mindestens ein Polysaccharid in einer Gesamtmenge von 0,01 - 1,0 Gew.-%, bevorzugt 0,05 - 0,5 und besonders bevorzugt 0,09 - 0,2 Gew.-%.

11. Kosmetische Antitranspirant-Zusammensetzung gemäß Anspruch 1, 2, 4, 7 oder 8, **dadurch gekennzeichnet, dass** sie, jeweils bezogen auf ihr Gesamtgewicht, enthält:
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,01 - 0,3 Gew.-%, bevorzugt 0,05 - 0,2 Gew.-%, besonders bevorzugt 0,1 - 0,15 Gew.-%, Squalan, weiterhin
c) insgesamt 0,01 - 0,5 Gew.-%, mindestens einer Substanz, ausgewählt aus Tocopherolen und Tocopherylestern sowie Mischungen hiervon, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%, der ausgewählt ist aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat sowie Mischungen hiervon,
e) 0 bis 6 Gew.-% freies Wasser,
f) insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, die bevorzugt ausgewählt ist aus n-Dodecansäure, n-Tridecansäure, n-Tetradecansäure, n-Pentadecansäure, n-Hexadecansäure, n-Heptadecansäure und n-Octadecansäure sowie Mischungen hiervon, bevorzugt ausgewählt ist aus n-Dodecansäure, n-Tetradecansäure, n-Hexadecansäure und n-Octadecansäure sowie Mischungen hiervon, besonders bevorzugt ausgewählt ist aus n-Hexadecansäure und n-Octadecansäure sowie Mischungen hiervon, außerordentlich bevorzugt aus einer Mischung von n-Hexadecansäure und n-Octadecansäure im Molverhältnis 1:1.

12. Kosmetische Antitranspirant-Zusammensetzung gemäß Anspruch 1, 2, 4, 7, 8 oder 11, **dadurch gekennzeichnet, dass** sie, jeweils bezogen auf ihr Gesamtgewicht, enthält:
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,01 - 0,3 Gew.-%, bevorzugt 0,05 - 0,2 Gew.-%, besonders bevorzugt 0,1 - 0,15 Gew.-%, Squalan, weiterhin
c) insgesamt 0,01 - 0,5 Gew.-%, mindestens einer Substanz, ausgewählt aus Tocopherolen und Tocopherylestern sowie Mischungen hiervon, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%, der ausgewählt ist aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat sowie Mischungen hiervon,
e) 0 bis 6 Gew.-% freies Wasser,
f) insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, die ausgewählt ist aus einer Mischung von n-Hexadecansäure und n-Octadecansäure im Molverhältnis 1:1.

13. Kosmetische Antitranspirant-Zusammensetzung gemäß Anspruch 1, 3, 5, 6 oder 10, **dadurch gekennzeichnet, dass** sie als Öl-in-Wasser-Emulsion, die keine Mikroemulsion darstellt, vorliegt und, jeweils bezogen auf ihr Gesamtgewicht, folgendes enthält: 60 bis 96 Gew.-%, bevorzugt 65 - 75 Gew.-%, freies Wasser, insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin 0,01 - 0,3 Gew.-%, bevorzugt 0,05 - 0,2 Gew.-%, besonders bevorzugt 0,1 - 0,15 Gew.-%, Squalan, weiterhin 0,01 - 0,5 Gew.-%, bevorzugt 0,1 - 0,4 Gew.-%, besonders bevorzugt 0,2 - 0,35 Gew.-%, außerordentlich bevorzugt 0,25 - 0,3 Gew.-% alpha-Tocopherylacetat, weiterhin insgesamt 0,1 - 5 Gew.-%, bevorzugt 0,2 bis 4,5 Gew.-%, besonders bevorzugt 0,3 bis 4 Gew.-%, außerordentlich bevorzugt 0,5 bis 3,5 Gew.-%, einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat, weiterhin Glycerin in einer Gesamtmenge von 0,01 bis 0,5 Gew.-%, bevorzugt 0,05 bis 0,3 Gew.-%, besonders bevorzugt 0,1 bis 0,2 Gew.-%, weiterhin Ca²⁺ in einer Gesamtmenge von 0,001 bis 0,1 Gew.-%, bevorzugt 0,01 bis 0,08 Gew.-%, besonders bevorzugt 0,03 bis 0,06 Gew.-%, außerordentlich bevorzugt 0,04 bis 0,05 Gew.-%, weiterhin 0,5 - 6,5 Gew.-% Öl- oder Fettphase, umfassend mindestens eine bei 20 °C flüssige Ölkomponente, ausgewählt aus PPG-15-Stearylether, weiterhin mindestens einen nichtionischen Emulgator mit einem HLB-Wert im Bereich von 3 - 6 in einer Gesamtmenge von 1,8 - 3 Gew.-%, ausgewählt aus Steareth-2, weiterhin mindestens einen nichtionischen Emulgator mit einem HLB-Wert im Bereich von 12 - 18 in einer Gesamtmenge von 1 - 2 Gew.-%, ausgewählt aus Steareth-21, und mindestens ein Polysaccharid in einer Gesamtmenge von 0,01 - 1,0 Gew.-%, bevorzugt 0,05 - 0,5 und besonders bevorzugt 0,09 - 0,2 Gew.-%.

14. Kosmetische Antitranspirant-Zusammensetzung gemäß Anspruch 1, 3, 5, 6, 10 oder 13, **dadurch gekennzeichnet, dass** sie als Öl-in-Wasser-Emulsion, die keine Mikroemulsion darstellt, vorliegt und, jeweils bezogen auf ihr Gesamtgewicht, folgendes enthält: 60 bis 96 Gew.-%, bevorzugt 65 - 75 Gew.-%, freies Wasser, insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin 0,1 - 0,15 Gew.-%, Squalan, weiterhin 0,25 - 0,3 Gew.-% alpha-Tocopherylacetat, weiterhin insgesamt 0,5 bis 3,5 Gew.-% einer Mischung aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat, weiterhin Glycerin in einer Gesamtmenge von 0,1 bis 0,2 Gew.-%, weiterhin Ca²⁺ in einer Gesamtmenge von 0,04 bis 0,05 Gew.-%, weiterhin 0,5 - 6,5 Gew.-% Öl- oder Fettphase, umfassend mindestens eine bei 20 °C flüssige Ölkomponente, ausgewählt aus PPG-15-Stearylether, weiterhin mindestens einen nichtionischen Emulgator mit einem HLB-Wert im Bereich von 3 - 6 in einer Gesamtmenge von 1,8 - 3 Gew.-%, ausgewählt aus Steareth-2, weiterhin mindestens einen nichtionischen Emulgator mit einem HLB-Wert im Bereich von 12 - 18 in einer Gesamtmenge von 1 - 2 Gew.-%, ausgewählt aus Steareth-21, und mindestens ein Polysaccharid in einer Gesamtmenge von 0,01 - 1,0 Gew.-%, bevorzugt 0,05 - 0,5 und besonders bevorzugt 0,09 - 0,2 Gew.-%.

15. Kosmetische Antitranspirant-Zusammensetzung gemäß Anspruch 1, 2, 4, 7, 8, 11 oder 12, **dadurch gekennzeichnet, dass** sie, jeweils bezogen auf ihr Gesamtgewicht, enthält:
a) insgesamt 3 - 30 Gew.-%, bezogen auf das Gesamtgewicht der kristallwasserfreien Aktivsubstanz (USP) in der Gesamtzusammensetzung, Antitranspirant-Wirkstoff(e), ausgewählt aus Aluminium-Salzen und Aluminium-Zirkonium-Salzen, weiterhin
b) insgesamt 0,1 - 0,15 Gew.-%, Squalan, weiterhin
c) insgesamt 0,01 - 0,5 Gew.-%, mindestens einer Substanz, ausgewählt aus Tocopherylacetat, sowie
d) mindestens einen Glycerindiester oder Glycerintriester von mindestens einer gesättigten, unsubstituierten Alkansäure mit 8 bis 18 Kohlenstoffatomen in einer Gesamtmenge von 0,1 - 5 Gew.-%, der ausgewählt ist aus Glycerindi-n-octanoat, Glycerindi-n-decanoat, Glycerindilaurat, Glycerindi-n-tetradecanoat, Glycerindipalmitat, Glycerindistearat, Glycerintri-n-octanoat, Glycerintri-n-decanoat, Glycerintrilaurat, Glycerintri-n-tetradecanoat, Glycerintripalmitat und Glycerintristearat sowie Mischungen hiervon,
e) 0 bis 6 Gew.-% freies Wasser,
f) insgesamt 0,05 bis 0,5 Gew.-%, bevorzugt 0,1 - 0,4 Gew.-%, besonders bevorzugt 0,2 - 0,3 Gew.-%, mindestens einer gesättigten, unsubstituierten Alkansäure mit 12 bis 18 Kohlenstoffatomen, die ausgewählt ist aus einer Mischung von n-Hexadecansäure und n-Octadecansäure im Molverhältnis 1:1.
